(19) Europäisches Patentamt
European Patent Office
Office européen des brevets

(11) Veröffentlichungsnummer: 0 162 217
A1

(12) # EUROPÄISCHE PATENTANMELDUNG

(21) Anmeldenummer: 85103011.4

(22) Anmeldetag: 15.03.85

(51) Int. Cl.⁴: C 07 D 249/08
C 07 D 401/04, A 61 K 31/41

(30) Priorität: 16.03.84 CH 1329/84

(43) Veröffentlichungstag der Anmeldung: 27.11.85 Patentblatt 85/48

(84) Benannte Vertragsstaaten: AT BE CH DE FR GB IT LI LU NL SE

(71) Anmelder: CIBA-GEIGY AG
Postfach
CH-4002 Basel(CH)

(72) Erfinder: Jaeggi, Knut A., Dr.
General Guisan-Strasse 44
CH-4054 Basel(CH)

(72) Erfinder: Heckendorn, Roland, Dr.
Blumenweg 20
CH-4144 Arlesheim(CH)

(74) Vertreter: Zumstein, Fritz sen., Dr. et al,
Bräuhausstrasse 4
D-8000 München 2(DE)

(54) **1,2,4-Triazacycloalkadienderivate.**

(57) 1,2,4-Triazacycloalkadienderivate der Formel

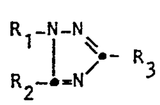

(I)

worin die Reste $R_1$ und $R_2$ unabhängig voneinander unsubstituierte oder durch aliphatische Kohlenwasserstoffreste, freies, veräthertes oder verestertes Hydroxy, gegebenenfalls S-oxidiertes veräthertes Mercapto, freies oder aliphatisch substituiertes Amino, Nitro und/oder Trifluormethyl substituierte Aryl- oder ggf. N-oxidierte Heteroarylgruppen bedeuten und $R_3$ einen unsubstituierten, mindestens 2 C-Atome aufweisenden oder durch eine Cyanogruppe, eine ggf. veresterte oder amidierte Carboxygruppe, eine oder zwei ggf. veresterte oder verätherte Hydroxygruppe(n), eine oder zwei verätherte Mercaptogruppe(n), eine oxidierte verätherte Mercaptogruppe oder eine Oxogruppe substituierten, ggf. durch Thia oder Oxa unterbrochenen aliphatischen oder einen unsubstituierten cycloaliphatischen Kohlenwasserstoffrest darstellt, und ihre Salze haben analgetische Eigenschaften und können als Arzneimittelwirkstoffe für die Behandlung schmerz-haft-entzündlicher Erkrankungen verwendet werden. Sie werden beispielsweise hergestellt, indem man aus einer Verbindung der Formel

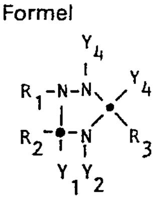

(II)

worin einer der Reste $Y_1$ und $Y_2$ einen abspaltbaren Rest Y und der andere ein Wasserstoffatom bedeutet und $Y_3$ und $Y_4$ gemeinsam eine zusätzliche Bindung darstellen, oder worin $Y_4$ einen abspaltbaren Rest Y und $Y_3$ Wasserstoff bedeutet und $Y_1$ und $Y_2$ eine zusätzliche Bindung darstellen, oder aus einem Salz davon unter Einführung einer zusätzlichen Bindung HY abspaltet.

EP 0 162 217 A1

0162217



CIBA-GEIGY AG
Basel (Schweiz)

4-14797/+

1,2,4-Triazacycloalkadienderivate

Die Erfindung betrifft neue 1,2,4-Triazacycloalkadienderivate der Formel

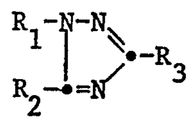

(I),

worin die Reste $R_1$ und $R_2$ unabhängig voneinander unsubstituierte oder durch aliphatische Kohlenwasserstoffreste, freies, veräthertes oder verestertes Hydroxy, gegebenenfalls S-oxidiertes veräthertes Mercapto, freies oder aliphatisch substituiertes Amino, Nitro und/oder Trifluormethyl substituierte Aryl- oder ggf. N-oxidierte Heteroarylgruppen bedeuten und $R_3$ einen unsubstituierten, mindestens 2 C-Atome aufweisenden oder durch eine Cyanogruppe, eine ggf. veresterte oder amidierte Carboxygruppe, eine oder zwei ggf. veresterte oder verätherte Hydroxygruppe(n), eine oder zwei verätherte Mercaptogruppe(n), eine oxidierte verätherte Mercaptogruppe oder eine Oxogruppe substituierten, ggf. durch Thia oder Oxa unterbrochenen aliphatischen oder einen unsubstituierten cycloaliphatischen Kohlenwasserstoffrest darstellt, und ihre Salze, Verfahren zur Herstellung der erfindungsgemässen Verbindungen, diese enthaltende pharmazeutische Präparate und ihre Verwendung.

Heteroarylreste sind beispielsweise monocyclische, ein Sauerstoff- oder Schwefelatom und gegebenenfalls zusätzlich ein Stickstoffatom aufweisende 5-gliedrige oder ein oder zwei Stickstoffatome aufweisende 6-gliedrige Heteroarylreste, wie Furyl, z.B. 2-Furyl, Thienyl,

z.B. 2-Thienyl, Thiazolyl, z.B. 2-Thiazolyl, ggf. N-oxidiertes Pyridyl, z.B. 2-, 4- oder insbesondere 3-Pyridyl bzw. 3-(1-Oxido-pyridyl), oder Pyrimidyl, z.B. 2-Pyrimidyl bzw. 2-(1-Oxido-pyrimidyl).

Arylreste sind Arylreste mit bis und mit 10 Kohlenstoffatomen (C-Atomen), beispielsweise monocyclische Arylreste, wie Phenyl.

Aryl- bzw. Heteroarylreste können können einen oder mehr als einen, z.B. ein, zwei oder drei, gleiche oder verschiedene der genannten Substituenten aufweisen.

Veräthertes Hydroxy ist dabei beispielsweise aliphatisch veräthertes Hydroxy, wie Niederalkoxy oder vicinal gebundenes Niederalkylendioxy bzw. Niederalkylidendioxy.

Verestertes Hydroxy als Substituent von $R_1$ bzw. $R_2$ ist z.B. mit einer Mineralsäure oder einer organischen Carbonsäure verestertes Hydroxy, wie Halogen oder Niederalkanoyloxy, ferner gegebenenfalls wie für Aryl angegeben, z.B. durch Niederalkyl, Niederalkoxy, Halogen und/oder Trifluoromethyl, substituiertes Benzoyloxy.

Gegebenenfalls oxidiertes veräthertes Mercapto als Substituent von $R_1$ bzw. $R_2$ ist z.B. Niederalkylthio, Niederalkansulfinyl oder Niederalkansulfonyl.

Aliphatisch substituierte Aminosubstituenten sind z.B. durch Niederalkyl N-mono- oder N,N-disubstituiertes Amino, wie Mono- oder Diniederalkylamino, oder in zweiter Linie 4- bis 7-gliedriges Alkylen- bzw. 3-Aza-, 3-Oxa- oder 3-Thia-alkylenamino.

Aliphatische Kohlenwasserstoffreste sind beispielsweise Niederalkylreste oder in zweiter Linie Niederalkenyl- oder Niederalkinylreste.

Als Substituenten, insbesondere von Niederalkyl, kommen beispielsweise Hydroxy, mit einer organischen Carbonsäure verestertes Hydroxy, z.B. Niederalkanoyloxy, oder aliphatisch veräthertes Hydroxy, z.B. Niederalkoxy, Niederalkylendioxy oder Niederalkylidendioxy, Niederalkylthio, Niederalkansulfinyl, Niederalkansulfonyl, Niederalkylendithio, Carboxy, Niederalkoxycarbonyl oder freies oder N-mono-, N,N-diniederalkyliertes oder durch 4- bis 7-gliedrige Alkylen bzw. 3-Aza-, 3-Oxa- oder 3-Thia-alkylen disubstituiertes Carbamyl, Cyano sowie Oxo in Betracht. Reste $R_3$ sind dementsprechend beispielsweise: Niederalkyl, ferner Niederalkenyl und Niederalkinyl, ebenso Mono- oder Dihydroxyniederalkyl, Mono- oder Diniederalkanoyloxyniederalkyl, Mono- oder Diniederalkoxyniederalkyl, Hydroxyniederalkoxyniederalkyl, Niederalkoxyniederalkoxyniederalkyl, Hydroxyniederalkylthioniederalkyl, Niederalkoxyniederalkylthioniederalkyl, Niederalkylendioxyniederalkyl, Niederalkylidendioxyniederalkyl, Mono- oder Diniederalkylthio-, Niederalkansulfinyl-, bzw. Niederalkansulfonylniederalkyl, Niederalkylendithioniederalkyl, Oxoniederalkyl, Carboxyniederalkyl, Niederalkoxycarbonylniederalkyl, Cyanoniederalkyl, Carbamylniederalkyl oder N-Mono- oder N,N-Diniederalkylcarbamylniederalkyl, ferner 4- bis 7-gliedriges N,N-Niederalkylen- bzw. N,N-(3-Aza-, 3-Oxa- oder 3-Thia)-niederalkylencarbamylniederalkyl.

Cycloaliphatische Kohlenwasserstoffreste sind beispielsweise monocycloaliphatische Kohlenwasserstoffreste mit 3 bis 8, insbesondere mit 5 bis 7 Ringgliedern, wie entsprechende Cycloalkylreste.

Vor- und nachstehend sind unter "niederen" organischen Resten und Verbindungen vorzugsweise solche zu verstehen, die bis und mit 7, vor allem bis und mit 4, Kohlenstoffatome (C-Atome) enthalten.

Niederalkyl $R_3$ ist beispielsweise über ein sekundäres oder tertiäres C-Atom gebundenes Niederalkyl mit 3 bis und mit 7 C-Atomen, wie

2-Propyl (Isopropyl), 2-Butyl (Sekundärbutyl), 2-(2-Methyl)-propyl (Tertiärbutyl), 2-Pentyl, 3-Pentyl, 2-(3-Methyl)-butyl, 2-(2-Methyl)-butyl, 2-Hexyl, 3-Hexyl, 2-(2,3-Dimethyl)-butyl, 3-Heptyl oder 3-(2,3-Dimethylpentyl oder, in zweiter Linie primäres Niederalkyl mit 2 bis und mit 7 C-Atomen, wie Aethyl, 1-Propyl, 1-Butyl, 1-Pentyl, 1-Hexyl oder 1-Heptyl. Anderes Niederalkyl als $R_3$ ist beispielsweise Methyl, Aethyl, Propyl, Isopropyl, Butyl, Isobutyl, Sekundärbutyl oder Tertiärbutyl, ferner Pentyl, Hexyl oder Heptyl.

Niederalkenyl ist beispielsweise Vinyl, vorzugsweise jedoch über ein gesättigtes C-Atom gebundenes Niederalkenyl, wie Allyl, Methallyl oder But-2-enyl.

Niederalkinyl ist beispielsweise Aethinyl, vorzugsweise jedoch über ein gesättigtes C-Atom gebundenes Niederalkinyl, wie Propargyl oder But-2-inyl.

Niederalkoxy ist beispielsweise Methoxy, Aethoxy, Propyloxy, Isopropyloxy, Butyloxy, Isobutyloxy, Sekundärbutyloxy oder Tertiärbutyloxy, ferner Pentyloxy, Hexyloxy oder Heptyloxy.

Niederalkylendioxy ist beispielsweise Methylendioxy, Aethylendioxy, 1,3-Propylendioxy, 2,3-Butylendioxy oder 1,3-(2,2-Dimethyl)-propylendioxy; Niederalkylidendioxy z.B. Aethylidendioxy oder Isopropylidendioxy.

Niederalkanoyloxy ist beispielsweise Acetoxy, Propionyloxy, Butyryloxy, Isobutyryloxy, ferner Formyloxy oder Pivaloyloxy.

Niederalkylthio ist beispielsweise Methylthio, Aethylthio, Propylthio, Isopropylthio, Butylthio, Isobutylthio, Sekundärbutylthio

oder Tertiärbutylthio, ferner Pentylthio, Hexylthio oder Heptylthio.

Niederalkylendithio ist beispielsweise Aethylendithio, 1,3-Propylendithio oder 1,3-(2,2-Dimethyl)-propylendithio.

Niederalkansulfinyl ist beispielsweise Methan-, Aethan-, 1- oder 2-Propan-, Butan- oder Isobutansulfinyl.

Niederalkansulfonyl ist beispielsweise Methan-, Aethan-, 1- oder 2-Propan-, Butan- oder Isobutansulfonyl.

Mono- oder Diniederalkylamino ist beispielsweise Methylamino, Dimethylamino, Aethylamino, Diäthylamino, Propylamino oder Butylamino.

4- bis 7-gliedriges Alkylenamino bzw. 3-Aza-, 3-Oxa- oder 3-Thiaalkylenamino ist beispielsweise Pyrrolidino, Piperidino, Morpholino, Thiomorpholino oder Piperazino bzw. 4-Methyl- oder 4-Aethylpiperazino.

4- bis 7-gliedriges N,N-Alkylen- bzw. N,N-(3-Aza-, 3-Oxa- bzw. 3-Thia)-alkylencarbamylniederalkyl ist beispielsweise Pyrrolidino-, Piperidino-, Morpholino-, Thiomorpholino- oder Piperazino- bzw. 4-Methyl- oder 4-Aethylpiperazinocarbonylmethyl.

Carboxyniederalkyl ist beispielsweise Carboxymethyl, 1- oder 2-Carboxyäthyl, 1-, 2- oder 3-Carboxypropyl, 2-(2-Carboxy)-propyl oder 4-Carboxybutyl, ferner 2-(2-Carboxy)-butyl.

Niederalkoxycarbonylniederalkyl ist beispielsweise Methoxycarbonylmethyl, Aethoxycarbonylmethyl, 1- oder 2-Methoxycarbonyläthyl, 1- oder 2-Aethoxycarbonyläthyl, 1-, 2- oder 3-Methoxycarbonylpropyl, 1-, 2- oder 3-Aethoxycarbonylpropyl, 2-(2-Methoxycarbonyl)- bzw. 2-(2-Aethoxycarbonyl)-propyl oder 4-Aethoxycarbonyl- bzw. 4-Methoxycarbonylbutyl, ferner 2-(2-Methoxycarbonyl)- bzw. 2-(2-Aethoxycarbonyl)-butyl.

Carbamylniederalkyl bzw. N-Mono- oder N,N-Diniederalkylcarbamylniederalkyl ist beispielsweise Carbamylmethyl, 1- oder 2-Carbamyläthyl oder 2-(2-Carbamyl)-propyl, N-Methyl-, N-Aethyl- oder N,N-Dimethylcarbamylmethyl, 1- oder 2-(N-Methyl-, N-Aethyl- oder N,N-Dimethylcarbamyl)-äthyl oder 2-[2-(N-Methyl-, N-Aethyl- oder N,N-Dimethylcarbamyl)]-propyl.

Cyanoniederalkyl ist beispielsweise Cyanomethyl, 1-Cyanoäthyl oder 2-(2-Cyano)-propyl.

Mono- oder Diniederalkoxyniederalkyl ist beispielsweise 2-Methoxy-, 2-Aethoxy-, 2-Propyloxy- oder 2-Isopropyloxy-, 2,2-Dimethoxy- oder 2,2-Diäthoxy-äthyl, 3-Methoxy- oder 3-Aethoxy-propyl oder 3,3-Dimethoxy-, 3,3-Diäthoxy-, 2,3-Dimethoxy- oder 2,3-Diäthoxypropyl oder 4,4-Dimethoxy-butyl, ebenso Methoxy-, Aethoxy-, Dimethoxy-, Propyloxy- oder Isopropyloxymethyl.

Niederalkylendioxyniederalkyl ist beispielsweise 2,2-Aethylendioxy- oder 2,2-Propylendioxy-äthyl oder 3,3- oder 2,3-Aethylendioxy-propyl, ebenso Aethylendioxy- oder Propylendioxymethyl.

Niederalkylidendioxyniederalkyl ist beispielsweise 2,3-Aethylidendioxy oder 2,3-Isopropylidendioxypropyl.

Mono- oder Diniederalkanoyloxyniederalkyl ist beispielsweise 1- oder 2-Acetoxyäthyl, 1- oder 2-Pivaloyloxyäthyl, 1-Acetoxypropyl-(2), 1-Pivaloyloxypropyl-(2), 1-Acetoxy-2-methyl-propyl-(2) oder 1-Pivaloyloxy-2-methyl-propyl-(2) oder 3-Acetoxy- oder 2,3-Diacetoxypropyl, kann aber auch Pivaloyloxymethyl oder Acetoxymethyl sein.

Mono- oder Diniederalkylthioniederalkyl bedeutet beispielsweise 2-Methylthio-, 2-Aethylthio-, 2-Propylthio oder 2-Isopropylthio-, 2,2-Dimethylthio- oder 2,2-Diäthylthio-äthyl, 3-Methylthio- oder

3-Aethylthio-propyl oder 3,3-Bis(methylthio)-, 3,3-Bis(äthylthio)-, 2,3-Bis(methylthio)- oder 2,3-Bis(äthylthio)-propyl oder 4,4-Bis-(methylthio)-butyl, ebenso Methylthio-, Aethylthio-, Bis(methylthio)-, Propylthio- oder Isopropylthio-methyl.

Niederalkansulfinylniederalkyl ist beispielsweise 2-Methansulfinyl-, 3-Aethansulfinyl-, 2-Propansulfinyl- oder 2-(2-Propansulfinyl)-äthyl, 3-Methansulfinyl- oder 2-Aethansulfinyl-propyl, ebenso Methansulfinyl-, Aethansulfinyl- oder 1- oder 2-Propansulfinyl-methyl.

Niederalkansulfonylniederalkyl ist beispielsweise 2-Methansulfonyl-, 3-Aethansulfonyl-, 2-Propansulfonyl- oder 2-(2-Propansulfonyl)-äthyl, 3-Methansulfonyl- oder 3-Aethansulfonyl-propyl, ebenso Methansulfonyl-, Aethansulfonyl- oder 1- oder 2-Propansulfonyl-methyl.

Niederalkylendithioniederalkyl ist beispielsweise 2,2-Aethylendithio- oder 2,2-Propylendithio-äthyl oder 3,3- oder 2,3-Aethylendithiopropyl, ebenso Aethylendithio- oder Propylendithio-methyl.

Mono- oder Dihydroxyniederalkyl ist beispielsweise Hydroxymethyl, 1- oder 2-Hydroxyäthyl, 3-Hydroxy- oder 2,3-Dihydroxy-propyl, 2-Hydroxypropyl-(2), 1-Hydroxy-2-methyl-propyl-(2), 4-Hydroxy- oder 2,4-Dihydroxybutyl, 5-Hydroxy-, 2,5-Dihydroxy- oder 3,5-Dihydroxypentyl.

Hydroxyniederalkoxyniederalkyl bedeutet beispielsweise 2-(2-Hydroxyäthoxy)-, 2-(3-Hydroxypropyloxy)- oder 2-(2,3-Dihydroxyäthoxy)-äthyl oder 3-(2-Hydroxyäthoxy)- oder 3-(3-Hydroxypropyloxy)-propyl, ebenso 2-Hydroxyäthoxy- oder 3-Hydroxypropyloxymethyl.

Niederalkoxyniederalkoxyniederalkyl bedeutet beispielsweise 2-(Methoxyäthoxy)-, 2-(Aethoxymethoxy)-, 2-(2-Methoxypropyloxy)- oder 2-(2-Aethoxyäthoxy)-äthyl oder 3-(3-Methoxyäthoxy)-propyl, ebenso Methoxyäthoxy-, Aethoxymethoxy- oder Aethoxyäthoxy-methyl.

Hydroxyniederalkylthioniederalkyl bedeutet beispielsweise 2-(2-Hydroxyäthylthio)-, 2-(3-Hydroxypropylthio)- oder 2-(2,3-Dihydroxypropylthio)-äthyl oder 3-(2-Hydroxyäthylthio)- oder 3-(3-Hydroxypropylthio)-propyl, ebenso 2-Hydroxyäthylthio- oder 3-Hydroxypropylthiomethyl.

Oxoniederalkyl bedeutet beispielsweise 2-Oxoäthyl, 2- oder 3-Oxopropyl oder 2-, 3- oder 4-Oxobutyl, ferner entsprechendes Oxopentyl, Oxohexyl oder Oxoheptyl oder Formyl.

Cycloalkyl mit 3 bis 8 Ringgliedern ist beispielsweise Cyclopentyl, Cyclohexyl oder Cycloheptyl, ferner Cyclopropyl oder Cyclooctyl.

Halogen ist beispielsweise Halogen der Atomnummer bis und mit 35, wie Fluor, Chlor oder Brom.

Salze von Verbindungen der Formel I sind vor allem pharmazeutisch verwendbare Salze, einerseits pharmazeutisch verwendbare Säureadditionssalze mit starken Säuren, wie Mineralsäure, z.B. Salze mit Halogenwasserstoffsäuren, vor allem Chlor- oder Bromwasserstoffsäure, d.h. Hydrohalogenide, vor allem Hydrochloride und Hydrobromide, oder Schwefelsäuresalze, d.h. Hydrogensulfate und Sulfate, sowie Salze mit geeigneten organischen Säuren, wie Dicarbonsäuren oder organischen Sulfonsäuren, z.B. Maleinate, Fumarate, Maleate,

Tartrate oder Methansulfonate, und N-Cyclohexylsulfaminate, andrerseits innere Salze von Verbindungen der Formel I, worin $R_3$ Carboxy aufweist, oder deren pharmazeutisch verwendbare Metall-, wie Alkali- oder Erdalkalimetallsalze oder Ammoniumsalze mit Ammoniak oder geeigneten organischen Aminen, wie Mono-, Di- oder Triniederalkylaminen, z.B. Diäthylamin, Mono-, Di- oder Trihydroxyniederalkylaminen, z.B. Triäthanolamin oder 2-Dimethylaminoäthanol, oder heteroaliphatischen Aminen, z.B. Morpholin.

Die Verbindungen der Formel I weisen wertvolle pharmakologische Eigenschaften auf. Insbesondere zeigen sie eine ausgeprägte antinociceptive Wirksamkeit sowie eine Hemmwirkung auf die Prostaglandinsynthese, ebenso eine deutliche antiinflammatorische Wirkung.
So erweisen sie sich an der Maus im durch Phenyl-p-benzochinon ausgelösten Writhing-Syndrom nach J. Pharmacol. exp. Therap. 125, 237 (1959) im Dosisbereich von etwa 4-40 mg/kg p.o. als ausgezeichnet wirksam.

Ebenso zeigen sie bei Dosen ab etwa 10 bis 100 mg/kg p.o. eine deutliche Hemmwirkung auf das experimentelle Carrageneen-Pfotenoedem der Ratte, Di Pasquale et al. Agents and Actions 5,256, 1975.

Ferner zeigen sie in vitro im Konzentrationsbereich von etwa 0,1 bis 1,5 µM/L eine deutliche Hemmwirkung auf die Prostaglandinsynthese aus Arachidonsäure, nachgewiesen in der Versuchsanordnung nach Prostaglandins 7,123 (1974).

Die Verbindungen der Formel I sind dementsprechend vorzüglich geeignet als Wirkstoffe von pharmazeutischen Präparaten zur Behandlung schmerzhaft-entzündlicher Erkrankungen, vor allem chronischer Schmerzzustände des rheumatischen Formenkreises, wie solcher der chronischen Arthritis.

Die Erfindung betrifft in erster Linie Verbindungen der Formel I, worin die Reste $R_1$ und $R_2$ unabhängig voneinander unsubstituierte oder durch Niederalkyl, Niederalkoxy bzw. an vicinalen C-Atomen Niederalkyl (id)endioxy, Hydroxy, Halogen, Niederalkanoyloxy, aber auch unsubstituiertes oder durch Niederalkyl, Niederalkoxy und/oder Halogen substituiertes Benzoyloxy, Niederalkylthio, Niederalkansulfinyl, Niederalkansulfonyl, Amino, Mono- oder Diniederalkylamino, ferner 4- bis 7-gliedriges Alkylen- bzw. 3-Aza-, 3-Oxa- oder 3-Thiaalkylenamino, Nitro und/oder Trifluormethyl substituierte 6 bis und mit 10 C-Atomen aufweisende Aryl- oder ein Sauerstoff- oder Schwefelatom und gegebenenfalls zusätzlich ein Stickstoffatom aufweisende 5-gliedrige bzw. gegebenenfalls N-oxidierte ein oder zwei Stickstoffatom(e) aufweisende 6-gliedrige Heteroarylreste bedeuten und $R_3$ Niederalkyl mit mindestens 2 C-Atomen, 3- bis 8-gliedriges Cycloalkyl, Mono- oder Dihydroxyniederalkyl, Mono- oder Diniederalkanoyloxyniederalkyl, Hydroxyniederalkoxyniederalkyl, Niederalkoxyniederalkoxyniederalkyl, Hydroxyniederalkylthioniederalkyl, Niederalkoxyniederalkylthioniederalkyl, Mono- oder Diniederalkoxyniederalkyl, Niederalkylendioxyniederalkyl, Niederalkylendithioniederalkyl, Mono- oder Diniederalkylthioniederalkyl, Niederalkansulfinylniederalkyl, Niederalkansulfonylniederalkyl, Oxoniederalkyl, Carboxyniederalkyl, Niederalkoxycarbonylniederalkyl, Cyanoniederalkyl, Carbamylniederalkyl, N-Mono- oder N,N-Diniederalkylcarbamylniederalkyl, ferner 4- bis 7-gliedriges N,N-Niederalkylen- bzw. N,N-(3-Aza-, 3-Oxa- oder 3-Thia)-niederalkylencarbamylniederalkyl darstellt, und ihre Salze.

Die Erfindung betrifft insbesondere Verbindungen der Formel I, worin die Reste $R_1$ und $R_2$ unabhängig voneinander unsubstituiertes oder durch Niederalkyl, Niederalkoxy, Hydroxy, Halogen, Niederalkanoyloxy, Niederalkylthio, Niederalkansulfonyl, Diniederalkylamino und/oder Trifluormethyl substituiertes Phenyl oder unsubstituiertes oder durch Niederalkyl, Niederalkoxy, Halogen oder Hydroxy substituiertes Furyl, wie 2-Furyl, Thienyl, wie 2-Thienyl, Pyridyl, wie 2-, 3- oder 4-Pyridyl,

bzw. 1-Oxidopyridyl, wie 2-, 3- oder 4-(1-Oxido)-pyridyl, bedeuten und $R_3$ Niederalkyl mit mindestens 2-C-Atomen, Mono- oder Dihydroxyniederalkyl, Mono- oder Diniederalkoxyniederalkyl, Niederalkylendioxyniederalkyl, Oxoniederalkyl, Niederalkylthioniederalkyl, Carboxyniederalkyl, Niederalkoxycarbonylniederalkyl, Cyanoniederalkyl, Carbamylniederalkyl, N-Mono- oder N,N-Diniederalkylcarbamylniederalkyl darstellt, und ihre Salze, insbesondere ihre pharmazeutisch verwendbaren Salze.

Die Erfindung betrifft vor allem Verbindungen der Formel I, worin die Reste $R_1$ und $R_2$ unabhängig voneinander unsubstituiertes oder durch Niederalkoxy mit bis und mit 4 C-Atomen, wie Methoxy, Niederalkylthio mit bis und mit 4 C-Atomen, wie Methylthio, oder Halogen der Atomnummer bis und mit 35, wie Fluor oder Chlor, substituiertes Phenyl, gegebenenfalls N-oxidiertes Pyridyl, wie 3-Pyridyl, oder unsubstituiertes Thienyl, wie 2-Thienyl, bedeuten und $R_3$ über ein sekundäres oder tertiäres C-Atom gebundenes Niederalkyl bis 3 bis und mit 7 C-Atomen, wie Isopropyl oder Tertiärbutyl, die Cyano- oder Niederalkylthiogruppe in α-Stellung tragendes Cyanoniederalkyl mit bis und mit 4 C-Atomen im Niederalkylteil, wie Cyanomethyl oder 2-Cyanopropyl-(2), oder Niederalkylthioniederalkyl mit jeweils bis und mit 4 C-Atomen, wie Alkylthiomethyl, bedeutet oder Hydroxyniederalkyl mit bis und mit 4 C-Atomen wie Hydroxyäthyl, Hydroxymethyl, 2-Hydroxypropyl-(2) oder 1-Hydroxy-2-methyl-propyl-(2), Niederalkanoyloxyniederalkyl mit bis und mit 7 C-Atomen im Niederalkanoyloxyteil und bis und mit 4 C-Atomen im Niederalkylteil, wie 1-Pivaloyloxy-2-methyl-propyl-(2), Oxoniederalkyl mit bis und mit 4 C-Atomen, wie Acetyl oder 2-Oxoäthyl, oder Diniederalkoxyniederalkyl bzw. Niederalkylendioxyniederalkyl mit jeweils bis und mit 4 C-Atomen in jedem Alkyl(en)-teil, wie 2,2-Dimethoxyäthyl,3,3-Dimethoxypropyl, Dimethoxymethyl oder 2,2-Aethylendioxyäthyl, bedeutet, und ihre, insbesondere pharmazeutisch verwendbaren Salze.

Die Erfindung betrifft ganz besonders Verbindungen der Formel I, worin $R_1$ und $R_2$ durch Niederalkoxy mit bis und mit 4 C-Atomen, wie Methoxy, substituiertes Phenyl bedeuten oder $R_1$ Pyridyl und $R_2$ Phenyl darstellt und $R_3$ über ein tertiäres C-Atom gebundenes Niederalkyl mit bis und mit 7 C-Atomen, wie Tertiärbutyl, die Carboxy-, Cyano-, Niederalkoxycarbonyl-, Niederalkylthio-, Hydroxyniederalkylthio-, Oxoniederalkylthio-, Diniederalkoxyniederalkylthio bzw. Niederalkylendioxyniederalkylthiogruppe in α-Stellung tragendes Carboxyniederalkyl, wie Carboxymethyl oder 1-Carboxyäthyl, Cyanoniederalkyl, wie Cyanomethyl oder 2-Cyanopropyl-(2), Niederalkoxycarbonylniederalkyl, wie 2-Aethoxycarbonylpropyl-(2), Niederalkylthioniederalkyl, wie Aethylthiomethyl, ω-Hydroxyniederalkylthioniederalkyl, ω-Oxoniederalkylthioniederalkyl, Diniederalkoxyniederalkylthioniederalkyl oder Niederalkylendioxyniederalkylthioniederalkyl mit insgesamt bis und mit 7 C-Atomen, wie 2-Hydroxyäthylthio-, 2-Oxoäthylthio-, 2,2-Dimethoxyäthylthio- oder 2,2-Aethylendioxyäthylthiomethyl, Hydroxyniederalkyl mit bis und mit 7 C-Atomen, wie 2-Hydroxypropyl-(2) oder 1-Hydroxy-2-methyl-propyl-(2), bedeutet, und ihre, insbesondere pharmazeutisch verwendbaren Salze.

Die Erfindung betrifft in allererster Linie Verbindungen der Formel I, worin $R_1$ und $R_2$ durch Niederalkoxy mit bis und mit 4 C-Atomen substituiertes Phenyl bedeuten und $R_3$ über ein tertiäres C-Atom gebundenes Niederalkyl mit bis und mit 7 C-Atomen, wie Tertiärbutyl, die Cyano-, Niederalkylthio- bzw. Diniederalkoxyniederalkylthiogruppe in α-Stellung tragendes Cyanoniederalkyl mit insgesamt bis und mit 7 C-Atomen, wie Cyanomethyl oder 2-Cyanopropyl-(2), Niederalkylthioniederalkyl mit insgesamt bis und mit 7 C-Atomen, wie Aethylthiomethyl, Diniederalkoxyniederalkylthioniederalkyl mit insgesamt bis und mit 7 C-Atomen oder Hydroxyniederalkyl mit bis und mit 7 C-Atomen wie 2-Hydroxypropyl-(2) oder 1-Hydroxy-2-methyl-propyl-(2)

bedeutet, und ihre, insbesondere pharmazeutisch verwendbaren Salze.

Die Erfindung betrifft namentlich die in den Beispielen genannten neuen Verbindungen der Formel I und ihre pharmazeutisch verwendbaren Salze, Verfahren zu ihrer Herstellung, diese enthaltende pharmazeutische Präparate und ihre Verwendung.

Die Verbindungen der Formel I können nach an sich bekannten Methoden hergestellt werden, beispielsweise indem man aus einer Verbindung der Formel

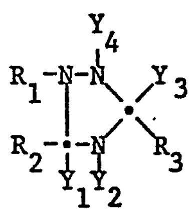

(II),

worin einer der Reste $Y_1$ und $Y_2$ einen abspaltbaren Rest Y und der andere ein Wasserstoffatom bedeutet und $Y_3$ und $Y_4$ gemeinsam eine zusätzliche Bindung darstellen, oder worin $Y_4$ einen abspaltbaren Rest Y und $Y_3$ Wasserstoff bedeutet und $Y_1$ und $Y_2$ eine zusätzliche Bindung darstellen, oder aus einem Salz davon unter Einführung einer zusätzliche Bindung HY abspaltet, erforderlichenfalls ein verfahrensgemäss erhältliches Isomerengemisch in die Komponenten auftrennt und das Isomere der Formel I isoliert und gewünschtenfalls eine verfahrensgemäss erhältliche Verbindung in eine andere Verbindung der Formel I umwandelt und/oder eine verfahrensgemäss erhältliche freie Verbindung in ein Salz oder ein verfahrensgemäss erhältliches Salz in die freie Verbindung oder in ein anderes Salz überführt.

Abspaltbare Reste Y sind beispielsweise gegebenenfalls veresterte oder verätherte Hydroxy- oder Mercaptogruppen, ferner Amino-, Ammonio- und Sulfoniumgruppen. Als verestertes Hydroxy kommt beispielsweise mit einer anorganischen Säure oder mit einer organischen Carbonsäure verestertes Hydroxy in Betracht, wie Halogen, z.B. Chlor oder Brom, oder Niederalkanoyloxy, z.B. Acetoxy. Verätherte Hydroxygruppen sind beispielsweise Niederalkoxygruppen, z.B. Methoxy oder Aethoxy. Veresterte Mercaptogruppen sind beispielsweise mit einer Niederalkancarbonsäure veresterte Mercaptogruppen, wie Acetylthio. Verätherte Mercaptogruppen bzw. Sulfoniumgruppen sind beispielsweise Niederalkylthio- bzw. Diniederalkylsulfoniumgruppen, wie Methylthio, Aethylthio oder Dimethylsulfonium. Aminogruppen sind neben Gruppen $R_1$-NH- beispielsweise Diniederalkyl- oder Alkylen- bzw. Aza-, Oxa- oder Thia-niederalkylenaminogruppen, z.B. Dimethylamino, Diäthylamino, Pyrrolidino, Piperidino, Morpholino oder Thiomorpholino, ferner Anilino. Ammoniumgruppen sind beispielsweise der vorstehend genannten Aminogruppe entsprechende tertiäre Ammoniumgruppen oder quaternäre Ammoniumgruppen, wie Triniederalkylammonio oder Pyridinio.

Die Abspaltung von HY erfolgt in üblicher Weise spontan oder durch gelindes Erwärmen, z.B. auf etwa 40 bis 100°C, erforderlichenfalls in Gegenwart eines Hilfsmittels und/oder eines inerten Lösungsmittels. Als Hilfsmittel kommen beispielsweise saure Mittel, wie Mineralsäuren oder deren Anhydride bzw. sauren Salze, z.B. Halogenwasserstoffsäuren, vor allem Chlor-, Brom- oder Jodwasserstoffsäure, Schwefelsäure, Alkalimetallhydrogensulfate, Phosphorsäure, Polyphosphorsäure, Phosphorpentoxid, Phosphortrichlorid, Phosphoroxychlorid oder Phosphortribromid, organische Sulfonsäuren, wie p-Toluolsulfonsäure, oder Carbonsäuren bzw. deren Anhydride, wie

Niederalkansäuren und deren Anhydride oder Halogenide, z.B. Essigsäure, Acetanhydrid oder Acetylchlorid, ferner gepufferte Säurelösungen, z.B. Phosphat- oder Acetatpuffer, Hydrohalogenide von Stickstoffbasen, z.B. Ammonium- oder Pyridiniumchlorid, in Betracht.

Vielfach kann man aber auch basische Kondensationsmittel, wie Hydroxide, Carbonate oder Niederalkanolate von Alkali- oder Eralkalimetallen, z.B. Natrium-, Kalium- oder Calciumhydroxid, Natrium- oder Kaliumcarbonat oder Natriummethanolat, verwenden, ferner organische Stickstoffbasen, wie Triniederalkylamine, z.B. Tiräthylamin, oder aromatische Tertiärbasen, wie Pyridin.

Die Ausgangsstoffe der Formel II werden vorzugsweise in situ hergestellt, beispielsweise indem man eine Verbindung der Formel

$$R_A - NH - C(Y_5)(Y_6) - R_B \qquad (III)$$

worin entweder $R_A$ eine Gruppe der Formel $R_1$-NH-N=C($R_3$)- und $R_B$ eine Gruppe $R_2$ oder $R_A$ eine Gruppe der Formel $R_2$-C(=NH)-N($R_1$)- und $R_B$ eine Gruppe $R_3$ darstellt und $Y_5$ und $Y_6$ unabhängig voneinander Reste Y, wie gegebenenfalls verestertes oder veräthertes Hydroxy, veräthertes Mercapto oder gegebenenfalls substituiertes Amino bedeuten oder gemeinsam Oxo, Thioxo oder gegebenenfalls substituiertes Imino, wie Niederalkylimino, gegebenenfalls substituiertes Anilo oder Gruppen der Formel =N-$R_1$, darstellen, oder ein Salz davon cyclisiert.

Die Cyclisierung von Verbindungen der Formel III erfolgt in üblicher Weise, unter neutralen, sauren oder basischen Bedingungen, erforderlichenfalls in Gegenwart eines der genannten sauren oder basischen Mittel, in Gegenwart oder Abwesenheit eines inerten

Lösungs- oder Verdünnungsmittels, unter Erwärmen, z.B. im Temperaturbereich von etwa 40-200°C, vorzugsweise von etwa 60-140°C, und/oder Inertgas, wie Stickstoff.

Für die Herstellung von Verbindungen der Formel III stehen mehrere Bildungsweisen zur Verfügung. So erhält man Verbindungen der Formel III, worin $R_A$ eine Gruppe der Formel $R_1$-NH-N=C($R_3$)- und $R_B$ eine Gruppe $R_2$ ist und $Y_5$ und $Y_6$ gemeinsam Oxo darstellen, beispielsweise, indem man eine Verbindung der Formel $R_1$-NH-N=C($R_3$)-$NH_2$ (IV), die beispielsweise durch Umsetzung einer Iminoverbindung der Formel Y-C(=NH)-$R_3$ (V), worin Y die angegebene Bedeutung hat und insbesondere Niederalkoxy, Halogen oder Amino bedeutet, oder eines Säureadditionssalzes, z.B. Hydrohalogenides, davon, z.B. in Aethanol, bzw. eines Triazinderivates der Formel

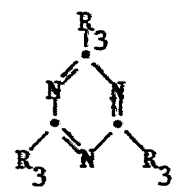

(VI)

mit einem substituierten Hydrazin der Formel $R_1$-NH-$NH_2$ (VII) oder durch Zersetzen eines 5-$R_3$-Tetrazols in Gegenwart eines Amins der Formel $R_1$-$NH_2$ (VIII) leicht zugänglich ist, oder ein Salz davon mit einer Verbindung der Formel Y'-C($Y_5$)($Y_6$)-$R_2$ (IX), worin $Y_5$ und $Y_6$ die angegebenen Bedeutungen haben und Y' eine Gruppe Y bzw., sofern $Y_5$ und $Y_6$ gemeinsam für Oxo stehen, eine Niederalkoxycarbonylmethyl-, Niederalkanoyloxy-, 1-Imidazolyl- oder eine Phthaliminooxygruppe oder eine Gruppe der Formel $R_2$-C(=O)-O- darstellt, in üblicher Weise, z.B. in Gegenwart eines basischen Kondensationsmittels, zur Reaktion bringt.

Man kann aber auch ein Amid der Formel $R_2$-C(=O)-$NH_2$ (X) mit einem Orthocarbonsäurederivat der Formel $R_3$-C($Y_5$)($Y_6$)-Y (XI, $Y_5$ = $Y_6$ = veräthertes Hydroxy bzw. Mercapto oder Halogen, Y =

disubstituiertes Amino, insbesondere Diniederalkylamino) zu einer Verbindung der Formel $R_2-C(Y_5')(Y_6')-N=C(Y)-R_3$ (XII, $Y_5'$ + $Y_6'$ = Oxo, Y = substituiertes Amino, z.B. Diniederalkylamino) umsetzen, z.B. in Dimethylformamid bei etwa 80 - 160° C, und dieses anschliessend unter sauren Bedingungen, z.B. in Essigsäure bei etwa 50 - 100° C, mit einem Hydrazin der Formel $R_1-NH-NH_2$ (VII) kondensieren.

Verbindungen der Formel III, worin $R_A$ eine Gruppe der Formel $R_2-C(=NH)-N(R_1)-$, $Y_5$ + $Y_6$ = Oxo und $R_B$ eine $R_3$-Gruppe ist, erhält man beispielsweise indem man eine Verbindung der Formel $R_2-C(=NH)-Y$ (XIII) bzw. ein Hydrohalogenid davon mit einer Verbindung der Formel $R_1-NH-NH-C(Y_5)(Y_6)-R_3$ (XIV), worin Y verestertes oder veräthertes Hydroxy, z.B. Halogen oder Niederalkoxy, bedeutet und $Y_5$ + $Y_6$ insbesondere für Oxo stehen, miteinander umsetzt, z.B. in einem Niederalkanol, wie Aethanol.

Im allgemeinen wird man jedoch auch die Zwischenstufe der Formel III nicht isolieren, denn die Synthese von Endstoffen der Formel I über Zwischenstufen der Formeln III und II kann gemäss einigen wichtigen Verfahrensvarianten ohne die Isolation von Zwischenprodukten durchgeführt werden. Einer wichtigen Verfahrensvariante zufolge kann man z.B. Verbindungen der Formeln $R_1-NH-NH_2$ (VII) und $R_2-C(Y_5')(Y_6')-NH-C(=O)-R_3$ (XV), worin $Y_5'$ + $Y_6'$ und Oxo oder gegebenenfalls substituiertes Imino der Formel $R_1-N=$, darstellt, miteinander umsetzen. Die Umsetzung erfolgt in üblicher Weise, beispielsweise in Gegenwart eines der genannten sauern oder basischen Kondensationsmittels, erforderlichenfalls in Gegenwart eines inerten Lösungs- oder Verdünnungsmittels, unter Erwärmen, z.B. im Temperaturbereich von etwa 40 - 200°C, vorzugsweise von etwa 60 - 140°C, und/oder unter Inertgas, wie Stickstoff.

Bevorzugte Ausführungsformen dieser über intermediär gebildete Zwischenstufen der Formeln III oder II verlaufenden direkten Synthesen von Verbindungen der Formel I sind die Umsetzung von Verbindungen der Formel VII mit Verbindungen der Formel XV ($Y_5$' und $Y_6$' = Oxo) unter schwach sauren Bedingungen, z.B. in Gegenwart einer organischen Carbonsäure, wie Essigsäure, eines Ammonium-Mineralsäuresalzes, z.B. von Pyridiniumchlorid in Essigsäure, oder eines sauren Puffersystems, wie eines Acetat- oder Phosphatpuffers, die Kondensation von Verbindungen der Formeln XIII und XIV, z.B. in Aethanol bei etwa 10 - 50°C, und anschliessender Cyclisierung bei etwa 80 - 160°C, z.B. in siedendem Xylol, ebenso Umsetzung eines Amidrazons der Formel IV mit einem Säureanhydrid der Formel IX (Y' z.B. Halogen, Niederalkanoyloxy oder $R_2COO$-; $Y_5 + Y_6$ = Oxo oder Thio) und anschliessende Basebehandlung, z.B. mit Natrium- oder Kaliumhydroxid.

Ebenfalls über Zwischenstufen der Formeln II und III verläuft die Umsetzung von Verbindungen der Formel $R_1$-NH-$NH_2$ (VII) mit einer Verbindung der Formel Y-C($Y_5$)($Y_6$)-$R_3$ (XI) und anschliessend mit einem Nitril der Formel $R_2$-CN (XVI). Einer anderen Variante zufolge kann man ein Amidrazon der Formel $R_1$-N($NH_2$)-C($R_2$)=NH (XVII) bei erhöhten Temperaturen, z.B. in der Schmelze bei etwa 100 - 230°C, z.B. bei etwa 190 - 210°C, oder in einem hochsiedenden organischen Lösungsmittel, z.B. in siedendem Xylol oder Dimethylformamid, mit einer Verbindung der Formel XV kondensieren. Auch dabei wird intermediär ein Zwischenprodukt der Formel II gebildet, das dann erfindungsgemäss unter HY-Abspaltung weiterreagiert.

Die neuen Verbindungen der Formel I können ferner hergestellt werden, indem man in einer Verbindung der Formel

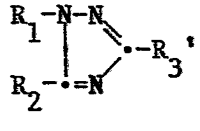

(XVIII) ,

worin $R_3$' einen in eine Gruppe $R_3$ überführbaren Rest bedeutet, $R_3$' in die gewünschte Gruppe $R_3$ überführt und erforderlichen- bzw. gewünschtenfalls eine oder mehrere der erwähnten Folgereaktionen durchführt.

In Reste $R_3$ überführbare Reste $R_3$' sind beispielsweise durch eine gegebenenfalls zusätzliche Carboxygruppe substituierte Reste $R_3$, insbesondere 1,1-Dicarboxyniederalkyl- oder 1-Carboxy-2-oxo-niederalkylreste oder Reste der Formel -C(=O)-O-$R_3$. Die Ueberführung dieser Reste in Reste $R_3$ erfolgt durch Abspaltung von Kohlendioxid.

Die Abspaltung von Kohlendioxid kann in üblicher Weise erfolgen, beispielsweise durch Säureeinwirkung, wie Behandlung mit einer Protonensäure, wie einer Mineralsäure, z.B. von Chlorwasserstoff- oder Schwefelsäure, vorteilhaft in einem Lösungs- oder Verdünnungsmittel, erforderlichenfalls unter Erwärmen, z.B. auf etwa 50 bis etwa 250°C. So kann man Verbindungen der Formel XVIII, worin $R_3'$ einen 1-Carboxy-2-oxo-niederalkylrest bedeutet, durch Erwärmen mit wässrigen Säurelösungen, z.B. mit gleichen Teilen etwa 15%-iger Salzsäure und Essigsäure auf etwa 60-100°C, in Verbindungen der Formel I überführen, worin $R_3$ einen 2-Oxoniederalkylrest bedeutet.

Ferner kann man Verbindungen der Formel XVIII worin $R_3$' einen Rest der Formel -C(=O)-O-$R_3$ darstellt, durch Erwärmen mit einer Mineralsäure, z.B. mit Chlorwasserstoff in Tetrahydrofuran, unter Kohlendioxidabspaltung in die entsprechenden Verbindungen der Formel I überführen.

Weitere in Gruppen $R_3$ überführbare Reste $R_3$' sind beispielsweise zu Resten $R_3$ reduzierbare Reste, wie gegebenenfalls in einer Ester-, Anhydrid- oder Salzform vorliegenden Carboxygruppen sowie 1 oder 2 mit einem α-Phenylniederalkanol verätherte Hydroxygruppe(n) oder

1 oder 2 mit einer halogenierten Niederalkansäure oder der Kohlensäure bzw. einem Halbester oder Halbamid derselben veresterte Hydroxygruppe(n) aufweisende Reste $R_3'$.

In einer Esterform vorliegende Carboxygruppen sind beispielsweise mit einem aliphatischen, cycloaliphatischen, araliphatischen oder aromatischen Alkohol veresterte Carboxygruppen, wie Niederalkoxycarbonyl, z.B. Methoxy- oder Aethoxycarbonyl, gegebenenfalls im Phenylteil substituiertes Benzoyloxymethoxycarbonyl, 2-Halogenniederalkoxycarbonyl, z.B. 2,2,2-Trichlor- oder 2-Jodäthoxycarbonyl, 3- bis 8-, insbesondere 5 bis 7-gliedriges Cycloalkoxycarbonyl, z.B. Cyclopentyloxycarbonyl oder Cyclohexyloxycarbonyl, gegebenenfalls substituierte Phenylniederalkoxycarbonyl-, insbesondere α-Phenylniederalkoxycarbonyl-, z.B. Benzyloxycarbonylreste oder gegebenenfalls substituiertes Phenyloxycarbonyl.

In einer Anhydridform vorliegendes Carboxy ist insbesondere mit einer Halogenwasserstoffsäure anhydridisierte Carboxygruppen, insbesondere Chlorcarbonyl.

In einer Salzform vorliegendes Carboxy liegt insbesondere in einer Alkalimetall- oder Erdalkalimetall- oder Ammoniumsalzform, z.B. als Natrium-, Kalium- oder Ammoniumsalz, vor.

1 oder 2 mit einem α-Phenylniederalkanol verätherte oder mit einer halogenierten Niederalkansäure oder mit Kohlensäure bzw. einem Halbester oder Halbamid derselben veresterte Hydroxygruppe(n) aufweisende aliphatische Kohlenwasserstoffrest weisen als Substituenten insbesondere Halogenniederalkanoyloxy, Niederalkoxycarbonyloxy, gegebenenfalls substituiertes Benzylcarbonyloxy, Diniederalkylcarbamyloxy oder Carbonyldioxy auf und bedeuten beispielsweise gegebenenfalls substituiertes Mono- oder Dibenzyloxyniederalkyl, Mono- oder Di(halogenniederalkanoyloxy)niederalkyl, Carbonyldioxy-

niederalkyl, Mono- oder Di(niederalkoxycarbonyloxy)niederalkyl oder gegebenenfalls substituiertes Benzyloxycarbonyloxyniederalkyl.

Als Phenylsubstituenten von gegebenenfalls substituiertes Phenyl aufweisenden Resten der genannten Art kommen z.B. Niederalkyl, Niederalkoxy, Halogen und/oder Nitro in Betracht.

Für die Reduktion von in einer Anhydridform vorliegendem Carboxy (zu Hydroxymethyl) sowie zur reduktiven Abspaltung von α-Phenylniederalkoxy- bzw. α-Phenylniederalkoxycarbonylgruppen kommt beispielsweise Wasserstoff in Gegenwart eines Hydrierungskatalysators, wie von Palladiumkohle, in Betracht.

Gegebenenfalls in einer Ester-, Anhydrid- oder Salzform vorliegende Carboxygruppen können ferner durch Umsetzung mit einem Hydridüberträger zu Hydroxymethyl bzw. durch Umsetzung mit einem Alkidüberträger zu α-Oxoniederalkylresten oder α-verzweigten α-Hydroxyniederalkylresten reduziert werden.

Als Hydridüberträger kommen beispielsweise Leichtmetall- oder Dileichtmetallhydride, wie Aluminiumhydrid, Lithiumaluminiumhydrid, Lithiumborhydrid, Lithium(triäthyl)borhydrid, für die Reduktion von Chlorcarbonyl ferner Natriumborhydrid, in Betracht. Geeignete Alkidüberträger sind beispielsweise Metallderivate, insbesondere Alkali- oder Erdalkaliderivate von Niederalkanen, wie Niederalkyl-, insbesondere Methyllithium, oder Niederalkylmagnesiumhalogenide. Für die Reduktion von Chlorcarbonyl zu α-Oxoniederalkyl besonders geeignet sind ferner Niederalkylzink- sowie -cadmiumhalogenide, die vorteilhaft aus dem entsprechenden Niederalkylmagnesiumhalogenid und Zink- bzw. Cadmiumhalogenid <u>in situ</u> hergestellt werden. Die Umsetzung mit den genannten Hydrid- bzw. Alkidüberträgern kann in üblicher Weise erfolgen, beispielsweise in einem aliphatischen oder cycloaliphatischen Aether, wie Di-

äthyläther, Tertiärbutyloxymethan, Tetrahydrofuran oder Dioxan, erforderlichenfalls unter Kühlen oder Erwärmen, vorteilhaft unter Inertgas.

2-Halogen-niederalkoxycarbonyloxy, z.B. 2,2,2-Trichlor- oder 2-Jod-äthoxycarbonyloxy, Benzoylmethoxycarbonyloxy oder 4-Nitrobenzyloxycarbonyloxy kann z.B. durch Behandeln mit einem geeigneten chemischen Reduktionsmittel, wie Zink in Gegenwart einer geeigneten Carbonsäure wie wässriger Essigsäure, abgespalten werden. Aroylmethoxycarbonyloxy kann auch durch Behandeln mit einem nucleophilen, vorzugsweise salzbildenden Reagens, wie Natriumthiophenolat, und 4-Nitrobenzyloxycarbonyloxy auch durch Behandeln mit einem Alkalimetall-, z.B. Natriumdithionit, abgespalten werden.

Weitere Reste $R_3'$ sind zu einer Gruppe $R_3$ solvolysierbare Reste, die beispielsweise 1 oder 2 Halogenatom(e), eine bivalente funktionell abgewandelte Oxogruppe, z.B. Imino, eine von verestertem und amidiertem Carboxy gemäss der eingangs gegebenen Definition sowie von Cyano verschiedene funktionell abgewandelte Carboxygruppe, wie eine Iminoäthergruppierung, eine gegebenenfalls wie für amidiertes Carboxy angegebene substituierte Amidinogruppierung, Halogencarbonyl oder gegebenenfalls substituiertes Phenoxycarbonyl, z.B. Phenoxy-, p-Nitrophenoxy- oder 2,4-Dinitrophenoxycarbonyl, oder mindestens eine von einer durch Halogen oder gegebenenfalls substituiertes Phenyl substituierten Alkancarbonsäure oder einem Kohlensäurehalbester abgeleitete Acyloxygruppe, mindestens eine Silyloxygruppe oder eine 1,2-Phenylendioxygruppe, zwei geminal gebundene Phenyloxygruppen oder eine vicinal gebundene Mono- oder Diphenylniederalkylidendioxy- bzw. Mono- oder Diphenylniederalkylidendithiogruppe aufweisen.

Abspaltbare Acyloxygruppen sind beispielsweise Halogenniederalkanoyloxy, wie 2-Halogenacetoxy, in 1-Stellung des Niederalkylrestes verzweigtes oder 1- oder 2-Stellung geeignet substituiertes Niederalkoxycarbonyloxy, insbesondere tert.-Niederalkoxycarbonyloxy, Arylmethoxycarbonyloxy mit einem oder zwei Phenylreste, die vorzugsweise gegebenenfalls durch Niederalkyl, insbesondere tert.-Niederalkyl, wie tert.-Butyl, Niederalkoxy, wie Methoxy, Hydroxy, Halogen, z.B. Chlor, und/oder Nitro, mono- oder polysubstituiertes Phenyl darstellen, wie gegebenenfalls substituiertes Benzyloxycarbonyloxy oder substituiertes Diphenylmethoxycarbonyloxy, Benzoylmethoxycarbonyloxy, worin die Benzoylgruppe vorzugsweise durch Halogen, wie Brom, substituiert ist. Solche Reste sind z.B. 2-Chlor-, 2-Brom-, 2-Jod-, 2,2,2-Trifluor- oder 2,2,2-Trichloracetoxy, tert.-Butyloxycarbonyloxy, 4-Nitrobenzyloxycarbonyloxy, Phenacyloxycarbonyloxy, 2-Halogenniederalkoxycarbonyloxy, z.B. 2,2,2-Trichloräthoxycarbonyloxy, 2-Bromäthoxycarbonyloxy oder 2-Jodäthoxycarbonyloxy, oder 2-Triniederalkylsilyläthoxycarbonyloxy, wie 2-Trimethylsilyläthoxycarbonyloxy oder 2-(Di-n-butyl-methyl-silyl)-äthoxycarbonyloxy, ferner 2-Triphenylsilyläthoxycarbonyloxy.

Eine Silyloxygruppe ist in erster Linie eine organische Silyloxygruppe, worin das Silicium vorzugsweise Niederalkyl, insbesondere Methyl, ferner Niederalkoxy, z.B. Methoxy, und/oder Halogen, z.B. Chlor, als Substituenten enthält. Entsprechende Silyloxygruppen sind in erster Linie Triniederalkylsilyloxy, insbesondere Trimethylsilyloxy, ferner Dimethyl-tert.-butyl-silyloxy, Niederalkoxyniederalkylhalogen-silyloxy, z.B. Methoxy-methyl-chlor-silyloxy, oder Diniederalkyl-halogensilyloxy, z.B. Dimethyl-chlor-silyloxy.

Mindestens eine der genannten Acyloxygruppen, mindestens eine Silyloxygruppe, eine 1,2-Phenylendioxygruppe, zwei geminal gebundene Phenyloxygruppen oder eine vicinal gebundene Mono- oder Diphenylniederalkylidendioxy- bzw. Mono- oder Diphenylniederalkylidendithiogruppe aufweisende Reste $R_3'$ können solvolytisch in Hydroxy aufweisende aliphatische Reste $R_3$, wie Mono- oder Dihydroxyniederalkyl, ein Halogenatom aufweisende Reste $R_3'$ ferner in durch veräthertes oder organisch verestertes Hydroxy oder veräthertes Mercapto substituierte aliphatische Reste $R_3$, wie Niederalkoxyniederalkyl, Niederalkanoyloxyniederalkyl oder Niederalkylthioniederalkyl, zwei

Halogenatome oder eine funktionell abgewandelte Oxogruppe aufweisende Reste $R_3'$ solvolytisch in Oxo aufweisende oder durch veräthertes oder organisch verestertes Hydroxy substituierte Reste $R_3$, wie Oxoniederalkyl, Diniederalkoxyniederalkyl oder Niederalkylendioxyniederalkyl, und eine funktionell abgewandelte Carboxygruppe aufweisende Reste $R_3'$ solvolytisch in gegebenenfalls verestertes oder amidiertes Carboxy $R_3$, wie Carboxyniederalkyl, Niederalkoxycarbonylniederalkyl oder gegebenenfalls aliphatisch N-substituiertes Carbamylniederalkyl überführt werden.

Die Solvolyse der zu Resten $R_3$ solvolysierbaren Gruppen $R_3'$ erfolgt beispielsweise durch Hydrolyse (Behandlung mit Wasser), Alkoholyse (Behandlung mit einem Alkohol), Mercaptolyse (Umsetzung mit einem Mercaptan), Umsetzung mit einer organischen Carbonsäure bzw. einem Salz davon oder Ammono- bzw. Aminolyse (Behandlung mit Ammoniak oder einem organischen Amin).

Durch Hydrolyse kann man beispielsweise mindestens eine der genannten Acyloxygruppen, mindestens eine Silyloxygruppe, eine 1,2-Phenylendioxygruppe, zwei geminal gebundene Phenyloxygruppen oder eine vicinal gebundene Mono- oder Diphenylniederalkylidendioxy- bzw. Mono- oder Diphenylniederalkylidendithiogruppe aufweisende Reste $R_3'$ in durch Hydroxy substituierte aliphatische $R_3$, wie Mono- oder Dihydroxyniederalkyl, zwei Halogenatome oder eine bivalente funktionell abgewandelte Oxogruppe aufweisende Reste $R_3''$ in Oxo aufweisende aliphatische Reste $R_3$, wie Oxoniederalkyl, funktionell abgewandeltes Carboxyniederalkyl $R_3'$ in Carboxyniederalkyl sowie eine Iminoäthergruppierung bzw. eine gegebenenfalls aliphatische N-substituierte Amidingruppierung aufweisende funktionell abgewandelte Carboxyniederalkylreste $R_3'$ in verestertes bzw. amidiertes Carboxyniederalkyl $R_3$ überführen.

Die Hydrolyse wird in üblicher Weise durchgeführt, erforderlichenfalls in Gegenwart eines Hydrolysemittels, und/oder eines inerten Lösungsmittels, unter Kühlen oder Erwärmen und/oder unter Inertgas. Hydrolysemittel sind beispielsweise saure oder alkalische Mittel. Saure Mittel sind beispielsweise wie Mineralsäuren, Halogenwasserstoffsäuren, z.B. Chlor-, Brom- oder Jodwasserstoffsäure, oder Sauerstoffsäuren des Schwefels oder Phosphors bzw. deren saure Salze, wie Schwefelsäure, Kaliumhydrogensulfat oder Phosphorsäure, oder organische Carbon- oder Sulfonsäuren, z.B. Niederalkansäure, wie Essigsäure, Trifluoressigsäure oder Chloressigsäure, oder p-Toluolsulfonsäure. Basische Mittel sind beispielsweise Hydroxide oder Carbonate von Alkali- oder Erdalkalimetallen, wie Kalium-, Natrium- oder Calciumhydroxid, bzw. Kalium- oder Natriumcarbonat. Inerte Lösungsmittel sind beispielsweise polare, mit Wasser mischbare Lösungsmittel, wie Alkohole, z.B. Methanol oder Aethanol, Niederalkylenäther, wie Dioxan, Diniederalkylketone, wie Aceton, tertiäre Amide, z.B. Dimethylformamid, N-Methylpyrrolidon, oder Hexamethylphosphorsäuretriamid, oder Diniederalkylsulfoxide, z.B. Dimethylsulfoxid.

Durch Alkoholyse können beispielsweise ein oder zwei Halogenatome oder bivalentes funktionell abgewandeltes Oxo aufweisende aliphatische Reste $R_3'$ in durch veräthertes Hydroxy substituierte aliphatische Kohlenwasserstoffreste $R_3$, wie Mono- oder Diniederalkoxyniederalkyl, Niederalkylendioxyniederalkyl, oder Halogencarbonyl aufweisende aliphatische Reste $R_3'$ in verestertes Carboxy, wie Niederalkoxycarbonyl, aufweisende Reste $R_3$ überführt und in Resten $R_3'$ mit organischen Carbonsäure veresterte Hydroxygruppen durch Umesterung freigesetzt werden. Die Alkoholyse erfolgt in üblicher Weise, beispielsweise in Gegenwart eines basischen, bei der Umesterung auch eines sauren Mittels, erforderlichenfalls unter Kühlen oder Erwärmen und/oder unter Inertgas, wie Stickstoff. Als saure bzw. basische Mittel kommen z.B. die genannten in Betracht, als basische Mittel ferner Metallalkoholate, wie Alkalimetall- oder Erdalkalimetallniederalkanolate, z.B. Natriummethanolat.

Durch Mercaptolyse kann man beispielsweise ein oder zwei Halogenatome oder bivalentes funktionell abgewandeltes Oxo aufweisende aliphatische Reste $R_3'$ in durch veräthertes Mercapto substituierte aliphatische Kohlenwasserstoffreste $R_3$, wie Niederalkthioniederalkyl, oder Hydroxyniederalkylthioniederalkyl, überführen. Die Mercaptolyse erfolgt in üblicher Weise, z.B. in Gegenwart eines basischen Kondensationsmittels oder indem man die Mercaptankomponente in Salzform, wie als Alkalimetallsalz, einsetzt.

Ein oder zwei Halogenatome aufweisende aliphatische Reste $R_3'$ können auch durch Umsetzung mit einem Salz einer Carbonsäure, wie einem Niederalkansäurealkalimetallsalz, in organisch verestertes Hydroxy aufweisende Reste $R_3$, wie Mono- oder Diniederalkanoyloxyniederalkyl, überführt werden.

Durch Amino- bzw. Aminolyse kann man beispielsweise Halogencarbonyl aufweisende aliphatischer Reste $R_3'$ in durch amidiertes Carboxy substituierte aliphatische Kohlenwasserstoffreste $R_3$, wie gegebenenfalls aliphatisch N-substituierte Carbamylniederalkylreste, überführen, vorteilhaft in Gegenwart eines basischen Kondensationsmittels. Man kann aber durch Aminolyse auch aus den genannten Acyloxygruppen die Hydroxygruppe freisetzen.

Halogenniederalkyl kann aber auch durch Umsetzung mit Cyanwasserstoffsäure oder einem Alkalimetallcyanid in Cyanoniederalkyl überführt werden. 2-substituiertes Silyläthoxycarbonyl lässt sich vorteilhaft durch Behandeln mit einem Alkalimetallfluorid, z.B. mit Kaliumfluorid, spalten.

Weiterhin kann $R_3'$ auch gegebenenfalls in Salzform, z.B. als Alkalimetallsalzform, vorliegendes Mercapto bedeuten, dass durch Umsetzung mit einem reaktionsfähigen Ester, beispielsweise einem Halogenwasserstoffsäureester, wie Chlor-, Brom- oder Jodwasserstoffsäureester, oder einem Sulfonsäureester, wie p-Toluolsulfonsäurester, eines gegebenenfalls durch 1 oder 2 freie, veresterte oder verätherte Hydroxygruppen, 1 oder 2 verätherte Mercaptogruppen oder oxidiertes veräthertes Mercapto substituierten aliphatischen Alkohols in einen gegebenenfalls entsprechend substituierten, durch Thia unterbrochenen aliphatischen Rest $R_3$ überführt werden kann.

Die Ausgangsstoffe können nach an sich bekannten Methoden hergestellt werden, beispielsweise durch Abspaltung von HY aus Verbindungen der Formel

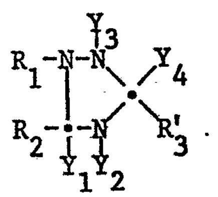

(XIX) ,

worin $Y_1$, $Y_2$, $Y_3$ und $Y_4$ die unter der Formel II angegebenen Bedeutungen haben.

Verbindungen der Formel XVIII, worin $R_3'$ gegebenenfalls in einer Ester-, Anhydrid- oder Salzform vorliegendes Carboxy bedeutet, können ferner hergestellt werden, indem man einen Aminomalonsäurediester in üblicher Weise mittels einer Verbindung der Formel $R_2-C(=O)-Cl$ (XX) N-acyliert,

das Reaktionsprodukt mit einem diazotierten Amin der Formel $R_1-NH_2$ (VIII) α-kuppelt und die erhaltene Azoverbindung durch Basebehandlung, z.B. Einwirkung von Natriummethanolat, unter Abspaltung einer veresterten Carboxygruppe cyclisiert. Aus dem erhaltenen Ester kann man dann hydrolytisch die freie Säure oder ein Salz davon und aus dieser mittels Thionylchlorid das Säurechlorid herstellen.

Die Verbindungen der Formel I können ferner hergestellt werden, indem man eine Verbindung der Formel

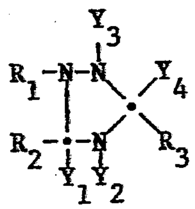

(II),

worin $Y_1$ und $Y_2$ Wasserstoff und $Y_3$ und $Y_4$ eine zusätzliche Bindung oder Wasserstoff oder $Y_1$ und $Y_2$ eine zusätzliche Bindung und $Y_3$ und $Y_4$ Wasserstoff bedeuten, dehydriert.

Die Dehydrierung erfolgt in üblicher Weise durch Behandlung mit einem dehydrierend wirkenden Oxidationsmittel, vorteilhaft in einem Lösungs- oder Verdünnungsmittel, erforderlichenfalls unter Kühlen oder Erwärmen, z.B. bei etwa 0 bis 200°C. Geeignete Oxidationsmittel sind beispielsweise Sauerstoff, wie Luftsauerstoff, erforderlichenfalls in Gegenwart eines Silber-, Mangan-, Eisen- oder Kobaltkatalysators, Perverbindungen, wie Wasserstoffperoxid, Metallperoxide, z.B. Nickelperoxid, Perkohlensäure und ihre Salze oder organische Persäuren, z.B. m-Chlorperbenzoesäure, Phthalmonopersäure oder Peressigsäure, oder ihre Salze, oxidierende Sauerstoffsäuren oder deren Salze oder Anhydride, wie unterhalogenige Säuren und ihre Salze, z.B. Natriumhypochlorit, Halogensäuren und ihre Salze, z.B. Jodsäuren, Perjodsäuren, Kaliumjodat, Natriumperjodat oder Kaliumchlorat, Salpetersäure bzw. salpetrige Säure und deren Salze und Anhydride, z.B. Kaliumnitrat, Natriumnitrit, Stickoxid, Distickstofftrioxid oder

Stickstoffdioxid, oder oxidierende Schwermetallverbindungen, wie Chrom-VI-, Chrom-IV-, Mangan-IV-, Mangan-VII-, Silber-II-, Kuper-II-, Quecksilber-II-, Vanadium-V- oder Wismut-II-verbindungen, z.B. Natriumdichromat, Kaliumdichromat, Chromtrioxid, Mangandioxid, Kaliumpermanganat, Silber-II-oxid, Kuper-II-oxid, Quecksilberoxid oder Wismutoxid. Inerte Lösungsmittel sind beispielsweise gegenüber dem jeweils zur Anwendung gelangenden Oxidationsmittel inerte Lösungsmittel, wie Wasser, Ketone, z.B. Aceton, Carbonsäuren, z.B. Essigsäure, Halogenkohlenwasserstoffe, aromatische, araliphatische oder heteroaliphatische Kohlenwasserstoffe, z.B. Tetrachlormethan, oder Aromaten oder Heteroaromaten, z.B. Benzol, Toluol, Xylol oder Pyridin, oder deren Gemische.

Die obigen Ausgangsstoffe der Formel II werden vorzugsweise in situ hergestellt, indem man unter oxidierenden Bedingungen wie unter Luftzutritt oder in Gegenwart von Eisen-III-chlorid, vorzugsweise in Xylol bei etwa 100°C bis Siedetemperatur eine Verbindung der Formel $R_1$-NH-N=C($R_3$)-$NH_2$ (IV), zugänglich beispielsweise durch Zersetzung eines 5-$R_3$-Tetrazols in Gegenwart eines Amins der Formel $R_1$-$NH_2$ (VIII) bzw. Kondensation von Verbindungen der Formeln $R_1$-NH-$NH_2$ (VII) und Y-C(=NH)-$R_3$ (V,Y = veräthertes Hydroxy), mit einer Verbindung der Formel $R_2$CH=O (XXI) kondensiert.

Verfahrensgemäss oder nach anderen vorstehend nicht erwähnten Verfahrensweisen erhältliche Verbindungen der Formel I können gewünschtenfalls in an sich bekannter Weise in andere Verbindungen der Formel I umgewandelt werden. Erforderlichenfalls kann ein verfahrensgemäss erhältliches Isomerengemisch in die reinen Isomeren aufgetrennt und gewünschtenfalls eine verfahrensgemäss erhältliche Verbindung in eine andere Verbindung der Formel I überführt und/oder eine verfahrensgemäss erhältliche freie Verbindung in ein Salz oder ein verfahrensgemäss erhältliches Salz in die freie Verbindung überführt werden.

So kann man beispielsweise gegebenenfalls verätherte oder veresterte Hydroxygruppen ineinander überführen.

Weist beispielsweise mindestens einer der Reste $R_1$, $R_2$ und $R_3$ eine Hydroxygruppe auf, so lässt sich diese auf üblichem Wege veräthern. So kann man Hydroxy als Bestandteil von $R_3$ mit einem Niederalkylierungsmittel, wie einem Niederalkanol, z.B. Methanol, in Gegenwart einer Säure, wie einer Mineralsäure, z.B. Schwefelsäure, oder eines Dehydratisierungsmittels, wie Dicyclohexylcarbodiimid, und Hydroxy an einem Rest $R_1$ bzw. $R_2$ z.B. in Gegenwart von Basen, wie Alkalimetallhydroxiden oder -carbonaten, z.B. Natriumhydroxid oder Kaliumcarbonat, mit Hilfe von Diniederalkylsulfaten oder Diazoniederalkanen in entsprechende Niederalkoxygruppen überführt. Umgekehrt lassen sich Aether, wie Niederalkoxygruppen als Substituenten von $R_1$ bzw. $R_2$ abspalten, beispielsweise durch Behandeln mit Säuren, wie mit Lewissäuren, z.B. Bortribromid oder Mineralsäuren, z.B. von Jodwasserstoff.

Weiterhin lässt sich Hydroxy verestern, z.B. in Niederalkanoyloxy umwandeln, beispielsweise durch Umsetzung mit einer entsprechenden Niederalkancarbonsäure, wie Essigsäure, oder einem reaktionsfähigen Derivat, wie einem symmetrischen Anhydrid, davon oder einem Anhydrid mit einer Halogenwasserstoffsäure, erforderlichenfalls in Gegenwart eines Kondensationsmittels, ausgehend von Anhydriden z.B. eines basischen Kondensationsmittels, wie eines Alkalimetallhydroxides oder -carbonats oder einer tertiären Stickstoffbase, z.B. eines Triniederalkylamins oder von Pyridin, und ausgehend von einer Säure, z.B. in Gegenwart einer Säure, wie einer Protonensäure, z.B. Chlorwasserstoff-, Schwefel-, Phosphor- oder einer Sulfonsäure, oder einer Lewissäure, z.B. Bortrifluorid-Etherat.

Ferner kann man freies Carboxyl als Bestandteil von $R_3$ in üblicher Weise, z.B. durch Behandeln mit einem Diazoniederalkan oder Triniederalkyloxonium-, Triniederalkylcarboxonium- oder Diniederalkylcarboniumsalz, wie Hexachloroantimonat oder Hexafluorophosphat, oder vor allem durch Umsetzung mit dem entsprechenden Alkohol oder einem reaktionsfähigen Derivat, wie einem Carbon-, Phosphorig-, Schweflig- oder Kohlensäureester, z.B. einem Niederalkancarbonsäureester, Triniederalkylphosphit, Diniederalkylsulfit oder dem Pyrocarbonat, oder einem Mineralsäure- oder Sulfonsäureester, z.B. dem Chlor- oder Bromwasserstoffsäure- oder Schwefelsäure-, Benzolsulfonsäure-, Toluolsulfonsäure- oder Methansulfonsäureester, des entsprechenden Alkohols oder einem davon abgeleiteten Olefin, zu einer veresterten Carboxylgruppe verestern.

Die Umsetzung mit dem entsprechenden Alkohol selbst kann vorteilhaft in Gegenwart eines sauren Katalysators erfolgen, wie einer Protonensäure, z.B. von Chlor- oder Bromwasserstoff-, Schwefel-, Phosphor-, Bor-, Benzolsulfon- und/oder Toluolsulfonsäure, oder eine Lewissäure, z.B. von Bortrifluorid-Aetherat, in einem inerten Lösungsmittel, insbesondere einem Ueberschuss des eingesetzten Alkohols und erforderlichenfalls in Gegenwart eines wasserbindenden Mittels und/oder unter destillativer, z.B. azeotroper, Entfernung des Reaktionswassers und/oder bei erhöhter Temperatur.

Die Umsetzung mit einem reaktionsfähigen Derivat des entsprechenden Alkohols kann in üblicher Weise durchgeführt werden, ausgehend von einem Carbon-, Phosphorig-, Schweflig- oder Kohlensäureester beispielsweise in Gegenwart eines sauren Katalysators, wie eines der vorstehend genannten, in einem inerten Lösungsmittel, wie einem aromatischen Kohlenwasserstoff, z.B. in Benzol oder Toluol, oder einem Ueberschuss des eingesetzten Alkoholderivates oder des entsprechenden Alkohols. Ausgehend von einem Mineralsäure- oder Sulfonsäureester setzt man die zu veresternde Säure vorteilhaft in Form eines Salzes,

z.B. des Natrium- oder Kaliumsalzes, und arbeitet erforderlichenfalls in Gegenwart eines basischen Kondensationsmittels, wie einer anorganischen Base, z.B. von Natrium- oder Kalium- oder Calciumhydroxid oder -carbonat, oder einer tertiären organischen Stickstoffbase, z.B. von Triäthylamin oder Pyridin, und/oder in einem inerten Lösungsmittel, wie einer der vorstehenden tertiären Stickstoffbasen oder eines polaren Lösungsmittels, z.B. in Dimethylformamid und/oder bei erhöhter Temperatur.

Cyano kann in üblicher Weise zu Carbamyl oder Carboxy hydrolysiert oder unter intermediärer Bildung einer Iminoäther- bzw. Amidingruppierung, die nachfolgend, auch ohne Isolierung, zu einer veresterten oder amidierten Carboxygruppe hydrolisiert werden kann, mit einem Alkohol bzw. Ammoniak oder einem primären oder sekundären Amin umgesetzt werden.

Eine freie Carboxylgruppe kann ferner durch Umsetzung mit Ammoniak oder einem mindestens ein Wasserstoffatom aufweisenden Amin in üblicher Weise, unter Dehydratisierung des intermediär gebildeten Ammoniumsalzes, z.B. durch azeotrope Destillation mit Benzol oder Toluol oder trockenes Erhitzen, in eine amidierte Carboxylgruppe überführt werden.

Die vorstehend beschriebenen Umwandlungen freier in veresterte oder amidierte Carboxylgruppen können aber auch so durchgeführt werden, dass man eine Verbindung der Formel I, worin $R_3$ Carboxy enthält, zunächst in üblicher Weise in ein reaktionsfähiges Derivat, beispielsweise mittels eines Halogenides des Phosphors oder Schwefels, z.B. mittels Phosphortrichlorid oder -bromid, Phosphorpentachlorid oder Thionylchlorid, in ein Säurehalogenid oder durch Umsetzung mit einem entsprechenden Alkohol oder Amin in einen reaktiven Ester, d.h. Ester mit elektronenanziehenden Strukturen, wie den Ester mit Phenol, Thiophenol, p-Nitrophenol oder Cyanmethylalkohol, oder ein reaktives

Amid, z.B. das von Imidazol oder 3,5-Dimethylpyrazol abgeleitete Amid, überführt und das erhaltene reaktionsfähige Derivat dann in üblicher Weise, z.B. wie nachstehend für die Umesterung bzw. gegenseitige Umwandlung veresterter und amidierter Carboxylgruppen beschrieben, mit einem entsprechenden Alkohol, Ammoniak oder dem entsprechenden mindestens ein Wasserstoffatom aufweisenden Amin zu der gewünschten Gruppe umsetzt.

Eine veresterte Carboxylgruppe, ebenso eine Trihalogenmethylgruppe, kann in üblicher Weise, z.B. durch Hydrolyse in Gegenwart eines Katalysators, beispielsweise eines basischen oder sauren Mittels, wie einer starken Base, z.B. von Natrium- oder Kaliumhydroxid, oder einer Mineralsäure, z.B. von Salzsäure, Schwefelsäure oder Phosphorsäure, in die freien Carboxylgruppe, verestertes Carboxy ferner z.B. durch Umsetzung mit Ammoniak oder dem entsprechenden, mindestens ein Wasserstoffatom aufweisenden Amin in eine amidierte Carboxylgruppe überführt werden.

Eine veresterte Carboxylgruppe kann ferner in üblicher Weise, z.B. durch Umsetzung mit einem Metallsalz, wie dem Natrium- oder Kaliumsalz, eines entsprechenden Alkohols oder mit diesem selbst in Gegenwart eines Katalysators, beispielsweise einer starken Base, z.B. von Natium- oder Kaliumhydroxid, oder einer starken Säure, wie einer Mineralsäure, z.B. von Salzsäure, Schwefelsäure, oder Phosphorsäure, oder einer organischen Sulfonsäure, z.B. von p-Toluolsulfonsäure, oder einer Lewissäure, z.B. von Brotrifluorid-Aetherat, zu einer anderen veresterten Carboxylgruppe umgeestert werden.

Eine amidierte Carboxylgruppe kann in üblicher Weise, z.B. durch Hydrolyse in Gegenwart eines Katalysators, beispielsweise einer starken Base, wie eines Alkalimetall- oder Erdalkalimetallhydroxides oder -carbonates, z.B. von Natrium- oder Kaliumhydroxid oder -carbonat, oder einer starken Säure, wie einer Mineralsäure, z.B. von Salzsäure, Schwefelsäure oder Phosphorsäure in die freie Carboxylgruppe

unsubstituiertes Carbamyl ferner durch Dehydratisierung, z.B. mittelts eines Carbodiimides oder Phosphorpentoxid bzw. Polyphosphor- oder Schwefelsäure, in Cyano umgewandelt werden.

Weiterhin kann man gegebenenfalls in einer Ester-, Anhydrid- oder Salzform vorliegende Carboxyniederalkylreste der Formel $-C_nH_{2n}-COOH$ in die Oxo- bzw. Hydroxygruppe in $\omega$-Stellung tragenden Oxoniederalkylreste der Formel $-C_{n+1}H_{2n+1}=O$ bzw. Hydroxyniederalkylreste der Formel $-C_{n+1}H_{2n+2}-OH$ reduzieren.

Die Reduktion erfolgt in üblicher Weise, beispielsweise durch Umsetzung mit einem Hydridüberträger, der auf das $\alpha$-ständige C-Atom Wasserstoff in anionischer Form überträgt, beispielsweise mit einem Leichtmetall- oder Dileichtmetallhydrides, z.B. mit Borhydrid-Aetherat oder vorzugsweise Lithiumaluminiumhydrid, vorteilhaft in einem Aether, wie einem Diniederalkyl- oder Niederalkylenäther, z.B. in Diäthyläther, Tertiärbutoxymethan oder Tetrahydrofuran, oder mittels Natriumborhydrid oder Natriumcyanoborhydrid, vorteilhaft in alkoholischer Lösung. Ebenso anwendbar sind Reduktionsmittel die nullwertige Metalle oder atomaren Wasserstoff übertragen. Dieser Prinzipien bedient man sich beispielsweise bei der metallischen Reduktion, die durch Einwirkung feinverteilter Metalle, z.B. von Zinkpulver, bewirkt werden kann, gleichermassen bei der Reduktion mit nascierendem Wasserstoff, der z.B. durch Säureeinwirkung auf unedle Metalle, wie von Essigsäure auf Zink, Eisen und Salzsäure oder Wassereinwirkung auf Natriumamalgam erzeugt werden kann. Durch die genannten Reduktionsmittel

erhält man vorzugweise Verbindungen der Formel I, worin $R_3$ einen Hydroxyniederalkylrest bedeutet. Salzformen von Carboxy sind dabei beispielsweise Basesalzformen, wie Alkali- oder Erdalkalimetallsalzformen, können aber auch Ammoniumsalzformen mit Ammoniak oder organischen Aminen sein. Anhydridformen sind beispielsweise gemischte Anhydridformen mit Halogenwasserstoffsäuren, können aber auch gemischte Anhydridformen mit organischen Carbonsäuren, wie Niederalkansäuren sein. Esterformen sind beispielsweise Niederalkylesterformen, können aber auch andere organische Esterformen, wie gegebenenfalls substituierte Phenylesterformen sein. Als weitere Esterformen kommen Lactonformen, z.B. γ-Lactonformen, in Betracht, bei deren Vorliegen in $\omega$-Stellung und in einer niederen als der ($\omega$-2)-Stellung zwei Hydroxygruppen aufweisende Reste $R_3$ gebildet werden. Die Reduktion kann aber auch auf der Oxostufe aufgehalten werden, indem man selektive Reduktionsmittel verwendet. Solche sind für die Reduktion von gegebenenfalls in Salzform vorliegendem Carboxy beispielsweise Mono- oder Diniederalkylborhydride, z.B. 2-(2,3-Dimethyl-butyl)-borhydrid, für die Reduktion von Anhydridformen beispielsweise Wasserstoff in Gegenwart von Palladium oder Cyanwasserstoffsäure in Gegenwart von tertiären aromatischen Stickstoffbasen, wie Chinolin, und für die Reduktion von Esterformen beispielsweise elektronegativ substituierte Aluminium- oder Alkalimetallaluminiumhydride, z.B. N-Methylpiperazino-aluminiumhydrid oder Natrium-bis-(2-methoxyäthoxy)-aluminiumhydrid, aber auch Dibutylaluminiumhydrid.

Als weitere Reduktionsmittel für die Reduktion von gegebenenfalls in einer Salz-, Anhydrid- oder Esterform vorliegenden Carboxy- zu Oxo- bzw. Hydroxygruppen kommen Alkidüberträger, die auf das α-ständige C-Atom einen Niederalkylrest in anionischer Form übertragen, z.B. Verbindungen von aliphatischen Kohlenwasserstoffen der

Formel $R_o$-H (XXVI) mit Metalle, z.B. Verbindungen der Formel $R_o-M^I$, $R_o-M^{II}$-Hal oder $(R_o)_2M^{II}$, worin $M^I$ ein Metallatom der Gruppe 1A und $M^{II}$ ein Metallatom der Gruppen 2A und 2B des Periodischen Systems der Elemente darstellt und $M^I$ vorzugsweise für Natrium oder Lithium und $M^{II}$ vorzugsweise für Magnesium oder Cadmium steht. Die Reduktion mit diesen Reduktionsmitteln, bei der in einer Salz-, Anhydrid- oder Esterform vorliegenden Carboxygruppen in tertiäre Hydroxygruppen überführt werden, erfolgt in üblicher Weise, beispielsweise in einem inerten Lösungsmittel, wie einen Diniederalkyl- oder Niederalkylenäther, z.B. in Diäthyläther oder Tetrahydrofuran, erforderlichenfalls unter Kühlen oder Erwärmen und/oder unter Inertgas wie Stickstoff. Auch hier kann die Reduktion von gegebenenfalls in einer Salz-, Anhydrid- oder Esterform vorliegenden Carboxyverbindung gewünschtenfalls auf der Oxostufe angehalten werden, indem man milde Reduktionsmittel, z.B. eine Cadmium- oder Halogencadmium-Verbindung bzw. eine Halogenmagnesiumverbindung in Gegenwart eines Cadmiumhalogenides, verwendet oder bei tiefen Temperaturen, z.B. unter -25°C, arbeitet.

Umgekehrt kann man in Verbindungen der Formel I Gruppen $R_3$ der Formel $-C_{n+1}H_{2n+2}-OH$ zu Gruppen $R_3$ der Formel $-C_{n+1}H_{2n+1}=O$ und Gruppen $R_3$ der Formeln $-C_nH_{2n}-CH_2OH$ bzw. $-C_nH_{2n}-CH=O$ zu solchen der Formel $C_nH_{2n}-COOH$ oxidieren.

Die Oxidation von Gruppen der Formel $-C_{n+1}H_{2n+2}-OH$ in solchen der Formel $-C_{n+1}H_{2n+1}=O$ erfolgt beispielsweise durch Behandeln mit einem für die Oxidation von primären und sekundären Alkoholen zu Aldehyden bzw. Ketonen üblichen Oxidationsmittel, wie nicht-oxidierenden Metallverbindungen, z.B. Chromylchlorid in Pyridin, Chromtrioxid in 2,5-Dimethylpyrazol, Nitrosoniumtetrafluorborat oder den Komplexen von Chlor bzw. Methansulfonsäureanhydrid und Dimethylsulfoxid, und weiteren, vor allem aber durch Behandeln mit einem aliphatischen oder cycloaliphatischen Keton, wie einem Oxoniederalkan, z.B. Aceton, oder einem

Cycloalkanon, z.B. Cyclohexanon, in Gegenwart eines Aluminiumalkoholates, z.B. von Aluminiumisopropanolat.

Die Oxidation von Gruppen der Formeln $-C_nH_{2n}-CH_2OH$ bzw. $-C_nH_{2n}-CH{=}O$ zu solchen der Formel $-C_nH_{2n}-COOH$ erfolgt beispielsweise durch Behandeln mit einer für die Oxidation von Alkoholen und Aldehyden üblichen Oxidationsmittel, wie oxidierenden Schwermetallverbindungen, z.B. Mangan-IV- und Mangan-VII-, Chrom-VI-, Blei-IV- oder Wismut-III-Verbindungen, insbesondere mit Kaliumpermanganat, Chromtrioxid oder Chromsäure bzw. Alkalimetallchromaten. Weiterhin geeignet ist Peroxyschwefelsäure, z.B. Caro'sche Säure, wobei bei Durchführung der Oxidation in einem Alkohol durch Veresterung der primär gebildeten Säure der entsprechende Ester erhalten wird.

Ferner können Verbindungen der Formel I, worin $R_3$ an vicinalen C-Atome veresterte Hydroxygruppen, wie Niederalkanoyloxy, aufweist, durch oxidative Acyloxylierung der Doppelbindung in entsprechenden nichtaromatische Doppelbindung(en) aufweisenden Verbindungen der Formeln I hergestellt werden. Die oxidative Acyloxylierung erfolgt beispielsweise durch Behandeln mit einem Schwermetallsalz einer organischen Carbonsäure, z.B. mit Bleitetraacetat, oder mit einem Silbersalz einer organischen Carbonsäure in Gegenwart von Halogen, z.B. mit Silberacetat in Gegenwart von Jod oder Brom.

Ferner kann man Verbindungen der Formel I, worin $R_3$ eine gegebenenfalls veresterte oder amidierte Carboxygruppe oder Cyano und in α-Stellung zu dieser mindestens ein Wasserstoff aufweist, durch Umsetzung mit einer tertiären Ammoniumbase, wie Tetrabutylammoniumhydroxid, oder einer Metallbase, wie einem Alkalimetall- oder Erdalkalimetallhydrid oder -amid, z.B. mit Natriumhydrid oder Natriumamid, α-deprotonieren und anschliessend mit einem reaktiven Ester eines

Niederalkanols, wie einem Niederalkylhalogenid, Niederalkylsulfonat, z.B. -p-toluolsulfonat, oder Diniederalkylsulfat, α-mono- oder gewünschtenfalls auch α,α-diniederalkylieren.

Ferner kann man in die Reste $R_1$ und/oder $R_2$ gegebenenfalls zusätzliche C-Substituenten einführen. So kann man in üblicher Weise halogenieren, z.B. durch Umsetzung mit Chlor oder Brom in Gegenwart von Eisen oder mittels N-Chlorsuccinimid. Ferner kann man in üblicher Weise, z.B. durch Umsetzung mit einem Alkylhalogenid, Alkanol oder Alken in Gegenwart von Aluminiumtrichlorid, alkylieren. Weiterhin kann man in üblicher Weise, z.B. mittels Salpetersäure/Schwefelsäure, nitrieren, die Nitrogruppe, z.B. mit Zinn-II-chlorid zu Amino reduzieren und diese mittels Natriumnitrit und Tetrafluorborsäure durch Fluor, mittels Salzsäure, Natriumnitrit und Kupfer-I-chlorid (-bromid) durch Chlor (Brom), bzw. mittels Natriumnitrit und Kaliumjodid durch Jod ersetzen oder mittels Natriumnitrit und eines Niederalkylmercaptans in Niederalkylthio bzw. mittels Natriumnitrit in Hydroxy überführen.

Ferner kann man in Oxo aufweisenden Resten $R_3$ Oxo zu Hydroxy reduzieren bzw. reduktiv durch Wasserstoff ersetzen, beispielsweise durch Einwirkung von nascierendem Wasserstoff, beispielsweise erzeugt durch Behandlung unedler Metalle mit Protonendonatoren, z.B. von Zink mit Salz- oder Essigsäure, von amalgamiertem Aluminium oder Natriumamalgam mit Wasser oder von Natrium mit einem Alkohol, wie Methanol. Der reduktive Ersatz von Oxo durch Wasserstoff kann auch erfolgen durch Umsetzung mit Hydrazin in Gegenwart einer Metallbase, z.B. von Natrium- oder Kaliumhydroxid oder eines Alkalimetallalkoholates, wie von Natriummethanolat in einem hochsiedenden Alkohol, wie Aethylenglykol, Diäthylenglykol oder Diäthylenglykolmonomethyläther, vorzugsweise bei erhöhter Temperatur, z.B. bei etwa 150 bis 250°C. Oxo kann ferner in üblicher Weise zu Hydroxy

reduziert werden, beispielsweise durch Umsetzung mit einem Leichtmetallhydrid, wie Boran-Tetrahydrofuran oder Diboran, oder einem Dileichtmetallhydrid, wie Natriumborhydrid, Natriumcyanoborhydrid oder Lithiumaluminiumhydrid, oder durch Umsetzung mit einem sekundären Alkohol, wie Isopropanol oder Cyclohexanol, in Gegenwart eines Aluminiumalkoholates. Die Reduktion von Oxo zu Hydroxy kann aber auch unter gleichzeitiger Einführung eines Kohlenwasserstoffrestes am Carbonylkohlenstoffatom erfolgen, indem man mit einer Niederalkylmetallverbindung, z.B. mit einem Niederalkyllithium oder Niederalkylmagnesiumhalogenid, umsetzt. In analoger Weise kann man durch Aldoladdition eines Ketons oder Aldehydes an eine aldehydische Oxogruppe unter Reduktion derselben zu Hydroxy einen in β-Stellung Oxo aufweisenden Rest einführen. Oxo kann ferner durch Behandlung mit einem Orthocarbonsäureester, z.B. einem Orthoameisensäureniederalkylester, oder unter wasserabspaltenden Bedingungen und Säurekatalyse mit einem Niederalkanol, Niederalkandiol, Niederalkylmercaptan bzw. Niederalkyldimercaptan, in Diniederalkoxy, Niederalkylendioxy, Diniederalkylthio bzw. Niederalkylendithio überführt werden.

Weiterhin kann man in Hydroxy aufweisenden Resten $R_3$ die Hydroxygruppe(n) reduktiv durch Wasserstoff ersetzen, beispielsweise wie oben für den reduktiven Ersatz von Oxo durch Einwirkung von Wasserstoff angegeben. Hydroxy kann man ferner durch Umsetzung mit einem Halogenierungsmittel, wie Thionylchlorid oder Phosphortribromid, bzw. einem Sulfonsäurechlorid, wie einem Alkan- oder gegebenenfalls substituierten Benzolsulfochlorid, z.B. mit p-Toluolsulfochlorid, , in Halogen bzw. Sulfonyloxy überführen, das dann durch Umsetzung mit einem Salz der Cyanwasserstoffsäure, wie Ammonium- oder einem Alkalimetallcyanid, durch Cyano oder durch Umsetzung mit einem unsubstituierten oder als Substituenten

eine Cyanogruppe, eine gegebenenfalls veresterte oder amidierte Carboxygruppe, eine oder zwei gegebenenfalls veresterte oder verätherte Hydroxygruppe(n), eine oder zwei verätherte Mercaptogruppe(n), eine oxidierte verätherte Mercaptogruppe oder eine Oxogruppe aufweisenden aliphatischen Alkohol oder Mercaptan bzw. einem Metallsalz davon durch den fehlenden Teil eines gegebenenfalls entsprechend substituierten, durch Oxa oder Thia unterbrochenen aliphatischen Kohlenwasserstoffrestes ersetzt werden kann. Man kann aber auch das Halogenatom durch Behandlung mit einem Alkali- oder Erdalkalimetall durch einen metallischen Rest, z.B. in den Lithium- oder einen Halogenmagnesiumrest, umwandeln, der dann durch Umsetzung mit einem reaktiven Kohlensäurederivat, wie Kohlendioxid, einem Halogen-, z.B. Chlorameisensäureester oder einem gegebenenfalls N-substituierten Carbamoylchlorid durch gegebenenfalls verestertes oder amidiertes Carboxy ersetzt werden kann. Ferner kann man Hydroxyalkyl $R_3$ durch Säurebehandlung unter Wasserabspaltung in Alkenyl überführen.

In den Resten $R_3$ kann man ferner geminal gebundenes Diniederalkoxy, Diniederalkylthio, Niederalkylendioxy und Niederalkylendithio zu Oxo bzw. vicinal gebundenes Niederalkylidendioxy aufweisendes Niederalkyl zu Dihydroxyniederalkyl hydrolysieren, vorzugsweise säurekatalytisch.

Ferner kann man verestertes Hydroxy, wie Niederalkanoyloxy oder gegebenenfalls substituiertes Benzoyloxy, aufweisende Reste $R_3$ zu den entsprechenden Hydroxy aufweisenden Resten hydrolysieren, z.B. wie vorstehend für die Hydrolyse von Verbindungen der Formel XVIII angegeben.

Werden in erfindungsgemässen Verfahren Verbindungen der Formel I enthaltende Isomerengemische erhalten, können diese, ebenso wie erfindungsgemäss erhältliche Stereoisomerengemische, in üblicher Weise in die Komponenten aufgetrennt werden.

So können derartige Isomerengemische, z.B. Gemische von Stereoisomeren bzw. Diastereomeren auf Grund der Unterschiede der physikalischen Eigenschaften der Komponenten durch übliche physikalische Trennverfahren, wie Kristallisations-, Chromatographie-, Destillations- oder Phasenverteilungsverfahren, in die Komponenten auftrennen.

Enantiomerengemische, wie Racemate, können durch Kristallisation aus optisch aktiven Lösungsmitteln, Chromatographie an optisch aktiven Feststoffen, Einwirkung von Mikroorganismen oder Umsetzung mit einer optisch aktiven Hilfsverbindung in Diastereomerengemische, z.B. mit einer optisch aktiven Säure in Gemische diastereomerer Säureadditionssalze, und Trennung derselben in die Diastereomeren aus denen die Enantiomeren in der jeweils üblichen Weise freigesetzt werden können in die Enantiomeren gespalten werden. Für diesen Zweck übliche optisch aktive Säuren sind z.B. D- oder L-Weinsäure, Di-o-Toluylweinsäure, Aepfelsäure, Mandelsäure, Camphersulfonsäuren oder Chinasäure.

Ferner können erhaltene freie Verbindungen in an sich bekannter Weise in Säureadditionssalze überführt werden, z.B. durch Umsetzen einer Lösung der freien Verbindung in einem geeigneten Lösungsmittel oder Lösungsmittelgemisch mit einer der vorgenannten Säuren oder mit einer Lösung davon, oder mit einem geeigneten Anionenaustauscher.

Erhaltene Säureadditionssalze können in an sich bekannter Weise in die freien Verbindungen umgewandelt werden, z.B. durch Behandeln eines basischen Endstoffes mit einer Säure bzw. eines sauren Endstoffes mit einer Base, wie einem Alkalimetallhydroxid, einem Metallcarbonat oder -hydrogencarbonat, oder Ammoniak, oder mit einem geeigneten Anionenaustauscher.

Erhaltene Salze können in an sich bekannter Weise in andere Salze überführt werden, z.B. durch Behandlung eines Salzes mit einem geeigneten Metallsalz, wie einem Natrium-, Barium- oder Silbersalz, in einem geeigneten Lösungsmittel, in welchem ein sich bildendes anorganisches Salz unlöslich ist und damit aus dem Reaktionsgemisch ausscheidet, in andere Säureadditionssalze übergeführt werden.

Die Verbindungen, einschliesslich ihrer Salze können auch in Form der Hydrate erhalten werden oder das zur Kristallisation verwendete Lösungsmittel einschliessen.

Infolge der engen Beziehung zwischen den neuen Verbindungen in freier Form und in Form ihrer Salze sind im vorausgegangenen und nachfolgend unter den freien Verbindungen oder ihren Salzen sinn- und zweckmässig gegebenenfalls auch die entsprechenden Salze bzw. freien Verbindungen zu verstehen.

Die Erfindung betrifft auch diejenigen Ausführungsformen des Verfahrens, bei denen man von einer auf beliebiger Verfahrensstufe als Zwischenprodukte erhältlichen Verbindungen ausgeht und die fehlenden Verfahrensschritte ausführt, oder einen Ausgangsstoff unter den Reaktionsbedingungen gebildet oder in Form eines Derivates davon, gegebenenfalls eines Salzes, verwendet.

Beim Verfahren der vorliegenden Erfindung werden vorzugsweise solche Ausgangsstoffe verwendet, welche zu den eingangs als besonders wertvoll geschilderten Verbindungen führen. Neue Ausgangsstoffe sowie Verfahren zu ihrer Herstellung bilden ebenfalls einen Gegenstand der vorliegenden Erfindung.

Bei den erfindungsgemässen pharmazeutischen Präparaten, welche Verbindungen der Formel I oder pharmazeutisch verwendbare Salze davon enthalten, handelt es sich um solche zur enteralen wie oralen oder rektalen, und parenteralen Verabreichung sowie zur topischen Anwendung an Warmblüter(n), welche den pharmakologischen Wirkstoff allein oder zusammen mit einem pharmazeutisch anwendbaren Trägermaterial enthalten. Die Dosierung des Wirkstoffs hängt von der Warmblüter-Spezies, dem Alter und dem individuellen Zustand, sowie von der Applikationsweise ab.

Im Normalfall ist für einen etwa 75 kg schweren Warmblüter bei oraler Applikation eine ungefähre Tagesdosis von etwa 50-500 mg, vorteilhaft in mehreren gleichen Teildosen verteilt, zu veranschlagen.

Die neuen pharmazeutischen Präparate enthalten z.B. von etwa 10 % bis etwa 80 %, vorzugsweise von etwa 20 % bis etwa 60 % des Wirkstoffs,Erfindungsgemäss pharmazeutische Präparate zur enteralen bzw. parenteralen Verabreichung sind z.B. solche in Dosiseinheitsformen wie Dragées, Tabletten, Kapseln oder Suppositorien, ferner Ampullen. Diese werden in an sich bekannter Weise, z.B. mittels konventioneller Misch-, Granulier-, Dragier-, Lösungs- oder Lyopilisierungsverfahren hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen, zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister unter Verwendung z.B. von Mais-, Weizen-, Reis- oder Kartoffelstärke, Gelatine, Traganth, Methylcellulose und/oder Polyvinylpyrrolidon, und/oder, wenn erwünscht, Sprengmittel, wie die obengenannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliessregulier- und Schmiermittel, z.B. Kieselsäure, Talk, Starisäure oder Salze davon, wie Magnesium- oder Calciumstearat, und/oder Polyäthylenglykol. Dragée-Kerne werden mit geeigneten, gegebenenfalls Magensaftresistenten Ueberzügen versehen, wobei man u.a. konzentrierte Zuckerlösungen, welche gegebenenfalls arabischen Gummi, Talk, Polyvinylpyrrolidon, Polyäthylenglycol und/oder Titandioxid enthalten, Lachlösungen in geeigneten organischen Lösungsmitteln oder Lösungsmittelgemischen oder, zur Herstellung von Magensaftresistenten Ueberzügen, Lösungen von geeigneten Cellulosepräparaten, wie Acetylcellulosephthalat oder Hydroxypropylmethylcellulosephthalat, verwendet.

Den Tabletten oder Dragée-Ueberzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbitol. Die Steckkapseln können den Wirkstoff in Form eines Granulats, z.B. im Gemisch mit Füllstoffen, wie Lactose, Bindemitteln, wie Stärken, und/oder Gleitmitteln, wie Talk oder Magnesiumstearat, und gegebenenfalls von Stabilisatoren, enthalten. In weichen Kapseln ist der Wirkstoff vorzugsweise in geeigneten Flüssigkeiten, wie fetten Oelen, Paraffinöl oder flüssigen Polyäthylenglycolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyäthylenglycole oder höhere Alkanole. Ferner können auch Gelatine-Rektalkapseln verwendet werden, die eine Kombination des Wirkstoffs mit einer Grundmasse enthalten; als Grundmassenstoffe kommen z.B. flüssige Triglyceride, Polyäthylenglykole oder Paraffinkohlenwasserstoff in Frage.

Zur parenteralen Verabreichung eigenen sich in erster Linie wässrige Lösungen eines Wirkstoffs in wasserlöslicher Form, z.B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Oele, z.B. Sesamöl, oder synthetische Fettsäureester, z.B. Aethyloleat oder Triglyceride, verwendet, oder wässrige Injektionssuspensionen, welche viskositätserhöhende Stoffe, z.B. Natrium-carboxymethylcellulose, Sorbit und/oder Dextran und gegebenenfalls auch Stabilisatoren enthalten.

Als topisch anwendbare pharmazeutische Präparate kommen in erster Linie Creme, Salben, Pasten, Schäume, Tinkturen und Lösungen, in Frage, die von etwa 0,5 bis etwa 20 % des Wirkstoffs enthalten.

Creme sind Oel-in-Wasser-Emulsionen, die mehr als 50 % Wasser aufweisen. Als ölige Grundlage verwendet man in erster Linie Fettalkohole, z.B. Lauryl-, Cetyl- oder Stearylalkohol, Fettsäuren, z.B. Palmitin- oder Stearinsäure, flüssige bis feste Wachse, z.B. Isopropylmyristat, Wollwachs oder Bienenwachs, und/oder Kohlenwasserstoffe, z.B. Vaseline (Petrolatum) oder Paraffinöl.Als Emulgatoren

kommen oberflächenaktive Substanzen mit vorwiegend hydrophilen Eigenschaften in Frage, wie entsprechende nichtionische Emulgatoren, z.B. Fettsäureester von Polyalkoholen oder Aethylenoxidaddukten davon, wie Polyglycerinfettsäureester oder Polyoxyäthylenfettalkoholäther oder -fettsäureester, oder entsprechende ionische Emulgatoren, wie Alkalimetallsalze von Fettalkoholsulfaten, z.B. Natriumlaurylsulfat, Natriumcetylsulfat oder Natriumstearylsulfat, die man üblicherweise in Gegenwart von Fettalkoholen, z.B. Cetylalkohol oder Stearylalkohol, verwendet. Zusätze zur Wasserphase sind u.a. Mittel, welche die Austrocknung der Creme vermindern, z.B. Polyalkohole, wie Glycerin, Sorbit, Propylenglykol und/oder Polyäthylenglykole, ferner Konservierungsmittel, Riechstoffe, etc.

Salben sind Wasser-in-Oel-Emulsionen, die bis zu 70 %, vorzugsweise jedoch von etwa 20 % bis etwa 50 % Wasser oder wässrige Phasen enthalten. Als Fettphase kommen in erster Linie Kohlenwasserstoffe, z.b. Vaseline, Paraffinöl und/oder Hartparafine in Frage, die zur Verbesserung des Wasserbindungsvermögens vorzugsweise geeignete Hydroxyverbindungen, wie Fettalkohole oder Ester davon, z.B. Cetylalkohol oder Wollwachsalkohole, bzw. Wollwachs enthalten. Emulgatoren sind entsprechende lipophile Substanzen, wie Sorbitanfettsäureester (Spans), z.B. Sorbitanoleat und/oder Sorbitanisostearat. Zusätze zur Wasserphase sind u.a. Feuchthaltungsmittel, wie Polyalkohole, z.B. Glycerin, Propylenglykol, Sorbit und/oder Polyäthylenglykol, sowie Konservierungsmittel, Riechstoffe etc.

Fettsalben sind wasserfrei und enthalten als Grundlage insbesondere Kohlenwasserstoffe, z.B. Paraffin, Vaseline und/oder flüssige Paraffine, ferner natürliche oder partialsynthetische Fett, z.B. Kokosfettsäuretriglycerid, oder vorzugsweise gehärtete Oele, z.B. hydriertes Erdnuss- oder Rizinusöl, ferner Fettsäurepartialester des Glycerins, z.B. Glycerinmono- und -distearat, sowie z.B. die im Zusammenhang mit den Salben erwähnten, die Wasseraufnahmefähigkeit steigernden Fettalkohole, Emulgatoren und/oder Zusätze.

Pasten sind Creme und Salben mit sekretabsorbierenden Puderbestandteilen, wie Metalloxiden, z.B. Titanoxid oder Zinkoxid, ferner Talk und/oder Aluminiumsilikate, welche die Aufgabe haben, vorhandene Feuchtigkeit oder Sekrete zu binden.

Schäume werden aus Druckbehältern verabreicht und sind in Aerosolform vorliegende flüssige Oel-in-Wasser-Emulsionen, wobei halogenierte Kohlenwasserstoffe, wie Chlorfluorniederalkane, z.B. Dichlordifluormethan und Dichlortetrafluoräthan, als Treibmittel verwendet werden. Als Oelphase verwendet man u.a. Kohlenwasserstoffe, z.B. Paraffinöl, Fettalkohole, z.B. Cetylalkohol, Fettsäureester, z.B. Isopropylmyristat, und/oder andere Wachse. Als Emulgatoren verwendet man u.a. Gemische von solchen mit vorwiegend hydrophilen Eigenschaften, wie Polyoxyäthylen-sorbitan-fettsäureester (Tweens), und solchen mit vorwiegend liphophilen Eigenschaften, wie Sorbitanfettsäureester (Spans). Dazu kommen die üblichen Zusätze, wie Konservierungsmittel, etc.

Tinkturen und Lösungen weisen meistens eine wässerig-äthanolische Grundlage auf, der u.a. Polyalkohole, z.B. Glycerin, Glyckole, und/oder Polyäthylenglykol, als Feuchthaltemittel zur Herabsetzung der Verdunstung, und rückfettende Substanzen, wie Fettsäureester mit niedrigen Polyäthylenglycolen, d.h. im wässrigen Gemisch lösliche, lipophile Substanzen als Ersatz für die der Haut mit dem Aethanol entzogenen Fettsubstanzen, und, falls notwendig, andere Hilfs- und Zusatzmittel beigegeben sind.

Die Herstellung der topisch verwendbaren pharmazeutischen Präparate erfolgt in an sich bekannter Weise, z.B. durch Lösen oder Suspendieren des Wirkstoffs in der Grundlage oder in einem Teil davon, falls notwendig. Bei Verarbeitung des Wirkstoffs als Lösung wird dieser in der Regel von der Emulgierung in einer der beiden Phasen gelöst; bei Verarbeitung als Suspension wird er nach der Emulgierung mit einem Teil der Grundlage vermischt und dann dem Rest der Formulierung beigegeben.

Die vorliegende Erfindung betrifft ebenfalls die Verwendung der Verbindungen der Formeln I und der Salze von solchen Verbindungen mit salzbildenden Eigenschaften, vorzugsweise zur Behandlung von Entzündungen, in erster Linie von entzündlichen chronischen Erkrankungen des rheumatischen Formenkreises, besonders der chronischen Arthritis.

Die nachfolgenden Beispiele illustrieren die oben beschriebene Erfindung; sie sollen jedoch diese in ihrem Umfang in keiner Weise einschränken. Temperaturen sind in Celsiusgraden, Drucke in mbar angegeben.

Beispiel 1: 5,5 g (0,0214 Mol) $N^1$-(p-Methoxyphenyl)-tertiärbutyramidrazon-hydrochlorid werden in 20 ml Wasser gelöst und mit 12 ml 2n-Natronlauge versetzt. Die freigesetzte Base wird mit Aether extrahiert. Der Extrakt wird mit Natriumsulfat getrocknet, filtriert und eingedampft. Das erhaltene Oel wird mit 20 ml Xylol (Isomerengemisch) und 2,75 g 4-Methoxybenzaldehyd unter Rückfluss 20 Stunden zum Sieden erhitzt. Dann wird unter vermindertem Druck eingedampft und der Rückstand aus Petroläther kristallisiert. Man erhält das 1,5-Bis-(p-methoxyphenyl)-3-tertiärbutyl-1H-1,2,4-triazol in Form farbloser Kristalle vom Smp. 101-102°.

Das als Ausgangsmaterial verwendete $N^1$-(p-Methoxyphenyl)-tertiärbutyramidrazon-hydrochlorid kann auf folgende Weise hergestellt werden:

7,6 g Pivaloimidsäuremethylesterhydrochlorid werden in 40 ml Methanol gelöst und mit einer Lösung von 7,0 g p-Methoxyphenylhydrazin in 100 ml Methanol versetzt. Nach 24 Stunden Stehenlassen bei 20° wird unter vermindertem Druck eingedampft. Der Rückstand liefert beim Anreiben mit Aether das $N^1$-(p-Methoxyphenyl)-tertiärbutyramidrazon-hydrochlorid, welches sich beim Erhitzen oberhalb 170° zersetzt.

Beispiel 2: Eine Lösung von 8,2 g (36 mMol) $N^1$-Phenyl-tertiärbutyramidrazon-hydrochlorid in 50 ml Methanol wird mit 6,66 ml einer 5,4-molaren Natriummethylatlösung in Methanol versetzt, das abgeschiedene Natriumchlorid abfiltriert und das Filtrat unter vermindertem Druck eingedampft. Der Rückstand wird mit 100 ml Xylol und 3,4 ml Pyridin-3-aldehyd 20 Stunden unter Rückfluss zum Sieden erhitzt.

Nach Abdestillieren des Xylols erhält man das 1-Phenyl-5-(3-pyridyl)-3-tertiärbutyl-1H-1,2,4-triazol in farblosen Kristallen vom Smp. 143-144° (aus Petroläther).

Das als Ausgangsmaterial verwendete $N^1$-Phenyl-tertiärbutyramidrazon-hydrochlorid kann auf folgende Weise erhalten werden:

Eine Lösung von 12,1 g Pivaloimidsäuremethylester-hydrochlorid in 100 ml Methanol wird mit 8,6 g Phenylhydrazin versetzt und 24 Stunden bei 20° aufbewahrt. Nach Eindampfen unter vermindertem Druck kristallisiert der Rückstand aus 2-Propanol. Man erhält das $N^1$-Phenyl-tertiärbutyramidrazon-hydrochlorid in farblosen Kristallen vom Smp. 237-239°.

Beispiel 3: 1,2 g (0,0052 Mol) $N^1$-(3-Pyridyl)-tertiärbutyramidrazon-hydrochlorid werden in 3 ml absolutem Methanol gelöst, mit 5,2 ml 1-n. Natriummethylatlösung in Methanol versetzt und vom ausgeschiedenen Natriumchlorid filtriert. Das Filtrat wird unter vermindertem Druck eingedampft, mit 20 ml Xylol (Isomerengemisch) und 0,6 g Benzaldehyd versetzt und anschliessend 20 Stunden unter Rückfluss zum Sieden erhitzt. Nach Abdestillieren des Xylols erhält man das 5-Phenyl-1-(3-pyridyl)-3-tertiärbutyl-1H-1,2,4-triazol in farblosen Kristallen vom Smp. 146-147° (aus Petroläther).

Das als Ausgangsmaterial verwendete $N^1$-(3-Pyridyl)-tertiärbutyramidrazon-hydrochlorid kann auf folgende Weise erhalten werden:

Eine Lösung von 9,6 g Pivaloimidsäuremethylesterhydrochlorid in 40 ml Methanol wird mit einer Lösung von 6,9 g 3-Hydrazinopyridin in 50 ml Methanol versetzt und 24 Stunden bei 20° aufbewahrt. Nach Eindampfen unter vermindertem Druck kristallisiert der Rückstand aus Aether. Man erhält das $N^1$-(3-Pyridyl)-tertiärbutyramidrazon-hydrochlorid vom Smp. 221-222°.

Beispiele 4: 20,5 g (0,0897 Mol) $N^1$-(3-Pyridyl)-tertiärbutyramidrazon-hydrochlorid werden in 150 ml Methanol gelöst, mit 16,6 ml einer 5,4-molaren Lösung von Natriummethylat in Methanol versetzt und vom ausgeschiedenen Natriumchlorid filtriert. Das Filtrat wird unter vermindertem Druck eingedampft, mit 200 ml Xylol (Isomerengemisch) und 12,2 g p-Methoxybenzaldehyd versetzt und anschliessend 20 Stunden unter Rückfluss zum Sieden erhitzt. Nach Abdestillieren des Xylols erhält man das 5-(p-Methoxyphenyl)-1-(3-pyridyl)-3-tertiärbutyl-1H-1,2,4-triazol in farblosen Kristallen vom Smp. 115-116° (aus Petroläther).

Beispiel 5: 15,2 g 1,5-Bis(p-methoxyphenyl)-1H-1,2,4-triazol-3-carbonsäuremethylester werden in einem Gemisch von 450 ml Tetrahydrofuran und 450 ml Methanol gelöst. Unter Rühren werden während 10 Minuten portionsweise 8,5 g Natriumborhydrid eingetragen. Nach 2 Stunden wird das Reaktionsgemisch mit 500 ml Wasser versetzt und unter vermindertem Druck von den organischen Lösungsmitteln befreit. Die verbleibende wässerige Lösung scheidet ein Oel aus, welches in Essigester aufgenommen wird. Die Essigester-Lösung wird über Natriumsulfat getrocknet, filtriert und eingedampft. Der Rückstand liefert aus Isopropanol das 1,5-Bis(p-methoxyphenyl)-1H-1,2,4-triazol-3-methanol vom Smp. 108-109°.

Das Ausgangsmaterial kann z.B. folgendermassen hergestellt werden:

70,3 g Aminomalonsäuredimethylester-hydrochlorid werden mit 65,4 g 4-Methoxybenzoylchlorid in 700 ml Methylenchlorid 48 Stunden unter Rühren und Rückfluss erhitzt. Dann wird abgekühlt, von Ungelöstem abfiltriert und das Filtrat mit Wasser und Kaliumbicarbonat-Lösung gewaschen. Die organische Phase wird über Natriumsulfat getrocknet, filtriert und eingedampft. Der Rückstand liefert aus Isopropanol den p-Methoxybenzoylaminomalonsäuredimethylester vom Smp. 114-115°.

2,0 g p-Methoxyanilin werden in einem Gemisch von 16 ml Eisessig und 4 ml konzentrierter Salzsäure gelöst, auf 0° abgekühlt und tropfenweise mit einer wässerigen 5-molaren Lösung von Natriumnitrit versetzt. Das Reaktionsgemisch wird auf -10° gekühlt und tropfenweise mit einer Lösung von 3,8 g p-Methoxybenzoylaminomalonsäuredimethylester in 40 ml Aceton versetzt. Dann wird eine Lösung von 22,8 g Kaliumcarbonat in 23 ml Wasser zugetropft und noch eine halbe Stunde bei 0-5° nachgerührt. Danach wird Essigester zum Reaktionsgemisch gegeben. Die organische Phase wird abgetrennt, mit Wasser gewaschen, über Natriumsulfat getrocknet, filtriert und eingedampft. Der Rückstand liefert aus Isopropanol den p-Methoxybenzoylamino-p-methoxyphenylazo-malonsäuredimethylester vom Smp. 139° (Zersetzung).

0,42 g p-Methoxybenzoylamino-p-methoxyphenylazo-malonsäuredimethylester werden in 9 ml Methanol suspendiert, mit 0,15 ml einer 1n-Natriummethylatlösung in Methanol versetzt und 3 Stunden bei 20° gerührt. Danach wird die erhaltene Lösung auf 0° abgekühlt, worauf Kristallisation erfolgt. Die Kristalle werden abgenutscht, mit Isopropanol gewaschen und getrocknet. Der so erhaltene 1,5-Bis(p-methoxyphenyl)-1H-1,2,4-triazol-3-carbonsäuremethylester schmilzt bei 174-176°.

Beispiel 6: 7,1 g (0,0216 Mol) 1,5-Bis(p-methoxyphenyl)-3-chlormethyl-1H-1,2,4-triazol werden mit einer Lösung von 4,6 g Aethylmercaptan in 80 ml 1n Natriummethylatlösung in Methanol 20 Stunden zum Rückfluss erhitzt. Die Reaktionslösung wird unter vermindertem Druck eingedampft und der Rückstand zwischen Wasser und Aether verteilt. Die Aetherphase wird mit Sole gewaschen, über Natriumsulfat getrocknet und eingedampft. Der Rückstand liefert aus Petroläther das 3-Aethylthiomethyl-1,5-bis(p-methoxyphenyl)-1H-1,2,4-triazol vom Smp. 89-90°.

Das als Ausgangsmaterial verwendete 1,5-Bis(p-methoxyphenyl)-3-chlormethyl-1H-1,2,4-triazol kann wie folgt hergestellt werden:

6,0 g 1,5-Bis(p-methoxyphenyl)-1H-1,2,4-triazol-3-methanol werden in 20 ml Chloroform gelöst und bei 10-15° zu einer Lösung von 6,0 ml Thionylchlorid in 10 ml Chloroform eingetropft. Nach 3 Stunden Aufbewahren bei 20° werden die flüchtigen Anteile unter vermindertem Druck bei 30 - 40° Badtemperatur abdestilliert. Der Rückstand ergibt nach Kristallisation aus Aether das 1,5-Bis(p-methoxyphenyl)-3-chlormethyl-1H-1,2,4-triazol vom Smp. 90-91°.

Beispiel 7

1,0 g 1,5-Bis(p-methoxyphenyl)-1H-1,2,4-triazol-3-methyl-S-thiuronium-chlorid werden in 10 ml Aethanol mit 2,9 ml 2-n. Natronlauge und 1,27 g 1-Brom-2,2-dimethoxyäthan 4 Stunden unter Rückfluss zum Sieden erhitzt. Dann lässt man das Aethanol abdestillieren und verteilt den Rückstand zwischen Wasser und Diäthyläther. Der Aetherextrakt wird mit Wasser gewaschen, über Natriumsulfat getrocknet und eingedampft. Mittels Kugelrohrdestillation erhält man das 1-[1,5-Bis(p-methoxyphenyl)-1H-1,2,4-triazol-3-ylmethylthio]-2,2-dimethoxy-äthan,
Kp. = 170°/2·$10^{-2}$ mbar, als farbloses Oel.

Das Ausgangsmaterial kann wie folgt hergestellt werden:

3,3 g 1,5-Bis(p-methoxyphenyl)-3-chlormethyl-1H-1,2,4-triazol werden in 15 ml Aethanol mit 0,84 g Thioharnstoff während 20 Stunden unter Rückfluss zum Sieden erhitzt. Beim Abkühlen erhält man das 1,5-Bis-(p-methoxyphenyl)-1H-1,2,4-triazol-3-methyl-S-thiuronium-hydrochlorid in farblosen Kristallen, Smp. 209-211°. (3,8 g = 94 % d.Th.).

Beispiel 8: 18,4 g Tetrabutylammoniumhydrogensulfat werden in 68 ml 2-n. Natronlauge gelöst und bei Raumtemperatur zu einer Lösung von 8,7 g 1,5-Bis(p-methoxyphenyl)-3-cyanomethyl-1H-1,2,4-triazol und 19,3 g Methyljodid in 50 ml Dichlormethan gegeben. Das Gemisch wird 20 Stunden bei Raumtemperatur gerührt. Anschliessend wird die Dichlormethan-Phase abgetrennt, mit Wasser gewaschen, über Natriumsulfat getrocknet, filtriert und eingedampft. Kristallisation des Rückstands aus Aethanol liefert das 2-[1,5-Bis(p-methoxyphenyl)-1H-1,2,4-triazol-3]-2-methyl-propionitril von Smp. 103 - 104°.

Beispiel 9: Zu einer mit Eis gekühlten Suspension von 5,7 g Natriumcyanid in 50 ml Dimethylsulfoxid werden unter Rühren 12,7 g 1,5-Bis-(p-methoxyphenyl)-3-chlormethyl-1H-1,2,4-triazol portionenweise zugegeben. Anschliessend wird das Reaktionsgemisch während 2 Stunden bei Raumtemperatur gerührt. Nach Zugabe von 350 ml Wasser wird mit Diäthyläther extrahiert. Die Extrakte werden mit Wasser gewaschen, über Natriumsulfat getrocknet, filtriert und eingedampft. Der Rückstand kristallisiert aus Diäthyläther. Das so erhaltene 2-[1,5-Bis(p-methoxyphenyl)-1H-1,2,4-triazol-3]-acetonitril schmilzt bei 76-78°.

Beispiel 10: 1,85 g Magnesium-Späne werden mit 10,6 g Methyljodid und 20 ml Diäthyläther behandelt. Nach Auflösung des Metalls wird eine Lösung von 10,2 g 1,5-Bis(p-methoxyphenyl)-1H-1,2,4-triazol-3-carbonsäuremethylester in 200 ml Tetrahydrofuran unter Rühren langsam zugetropft und anschliessend das Reaktionsgemisch 2 Stunden auf 50° erwärmt. Dann wird das Reaktionsgemisch auf 0° abgekühlt und tropfenweise mit 100 ml gesättigter Ammoniumchlorid-Lösung versetzt. Es bilden sich zwei Phasen, von welchen die obere abgetrennt und mit gesättigter Ammoniumchlorid-Lösung gewaschen wird. Anschliessend trocknet man die Tetrahydrofuran-Lösung über Natriumsulfat und dampft unter vermindertem Druck ein. Kristallisation des Rückstands aus Aether-Hexan liefert das 2-[1,5-Bis(p-methoxyphenyl)-1H-1,2,4-triazol-3]-propan-2-ol vom Smp. 96 - 97°.

Beispiel 11: 2,75 g (0,0107 Mol) $N^1$-(p-Methoxyphenyl)-tertiärbutyramidrazon und 10 g p-Methoxybenzoylchlorid werden unter Rühren 12 Stunden auf 120° erhitzt. Danach wird das Reaktionsgemisch mit überschüssiger 10 %iger Natriumcarbonatlösung verrührt, bis alles überschüssige Säurechlorid zersetzt ist. Man extrahiert mehrmals mit Diäthyläther, trocknet die Extrakte über Natriumsulfat, filtriert und dampft das Filtrat ein. Man erhält das 1,5-Bis(p-methoxyphenyl)-3-tertiärbutyl-1H-1,2,4-triazol vom Smp. 101 - 102°.

Analog erhält man aus 1,2 g $N^1$-(3-Pyridyl)-tertiärbutyramidrazon das 5-(p-Methoxyphenyl)-1-(3-pyridyl)-3-tertiärbutyl-1H-1,2,4-triazol vom Smp. 115 - 116°, sowie durch Umsetzung mit der entsprechenden Menge Benzoylchlorid, das 5-Phenyl-1-(3-pyridyl)-3-tertiärbutyl-1H-1,2,4-triazol vom Smp. 146 - 147°.

Beispiel 12: In analoger Weise wie in den Beispielen 1 bis 11 beschrieben kann man ferner herstellen:

2-[1,5-Bis(p-methoxyphenyl)-1H-1,2,4-triazol-3]-2-methyl-propionsäureäthylester, Smp. 106-107°,
2-[1,5-Bis(p-methoxyphenyl)-1H-1,2,4-triazol-3]-2-methyl-propan-1-ol, Smp. 109 - 110°, und
2-[1,5-Bis(p-methoxyphenyl)-1H-1,2,4-triazol-3-yl]-2-methyl-1-pivaloyloxy-propan, Smp. 90-92°.

Beispiel 13: Tabletten, enthaltend 25 mg Wirkstoff, z.B. 5-Phenyl-1-(3-pyridyl)-3-tertiärbutyl-1H-1,2,4-triazol, können folgendermassen hergestellt werden:

Bestandteile (für 1000 Tabletten):

| | |
|---|---|
| Wirkstoff | 25,0 g |
| Lactose | 100,7 g |
| Weizstärke | 7,5 g |
| Polyäthylenglykol 6000 | 5,0 g |
| Talkum | 5,0 g |
| Magnesiumstearat | 1,8 g |
| entmineralisiertes Wasser | q.s. |

Herstellung: Sämtliche festen Ingredienzien werden zunächst durch ein Sieb mit 0,6 mm Maschenweite getrieben. Dann wird der Wirkstoff, die Lactose, das Talkum, das Magnesiumstearat und die Hälfte der Stärke vermischt. Die andere Hälfte der Stärke wird in 40 ml Wasser suspendiert und diese Suspension zu einer siedenden Lösung des Polyäthylenglykols in 100 ml Wasser hinzugegeben und das Gemisch, wenn nötig unter Hinzufügen von Wasser, granuliert. Das Granulat wird über Nacht bei 35° getrocknet, durch ein Sieb mit 1,2 mm Maschenweite getrieben und zu beidseitig konkaven Tabletten von etwa 6 mm Durchmesser verpresst.

In analoger Weise können auch Tabletten, enthaltend jeweils 25 mg einer der in den Beispielen 1, 2 und 4 bis 11 genannten Verbindungen der Formel I hergestellt werden, wobei diese Verbindungen auch in Form von Salzen vorliegen können.

Beispiel 14: Kautabletten, enthaltend 30 mg Wirkstoff, z.B. 5-Phenyl-1-(3-pyridyl)-3-tertiärbutyl-1H-1,2,4-triazol, können z.B. folgendermassen hergestellt werden:

Zusammensetzung (für 1000 Tabletten):

| | |
|---|---|
| Wirkstoff | 30,0 g |
| Mannit | 267,0 g |
| Lactose | 179,5 g |
| Talkum | 20,0 g |
| Glycin | 12,5 g |
| Stearinsäure | 10,0 g |
| Saccharin | 1,0 g |
| 5%-ige Gelatinelösung | q.s. |

Herstellung: Alle festen Ingredienzien werden zunächst durch ein Sieb mit 0,25 mm Maschenweite getrieben. Der Mannit und die Lactose werden

gemischt, unter Hinzufügen von Gelatinelösung granuliert, durch ein Sieb mit 2 mm Maschenweite getrieben, bei 50° getrocknet und nochmals durch ein Sieb mit 1,7 mm Maschenweite getrieben. Der Wirkstoff, das Glycin und das Saccharin werden sorgfältig vermischt, der Mannit, das Lactosegranulat, die Stearinsäure und das Talkum hinzugegeben, das Ganze gründlich vermischt und zu beidseitig konkaven Tabletten von etwa 10 mm Durchmesser mit Bruchrille auf der Oberseite verpresst.

In analoger Weise können auch Tabletten enthaltend jeweils 30 mg einer der in den Beispielen 1, 2 und 4 bis 11 genannten Verbindungen der Formel I hergestellt werden, wobei diese Verbindungen auch in Form von Salzen vorliegen können.

Beispiel 15: Tabletten enthaltend 100 mg Wirkstoff, z.B. 5-Phenyl-1-(3-pyridyl)-3-tertiärbutyl-1H-1,2,4-triazol können z.B. folgendermassen hergestellt werden:

Zusammensetzung (für 1000 Tabletten):

| | |
|---|---|
| Wirkstoff | 100,0 g |
| Lactose | 248,5 g |
| Maisstärke | 17,5 g |
| Polyäthylenglykol 6000 | 5,0 g |
| Talkum | 15,0 g |
| Magnesiumstearat | 4,0 g |
| entmineralisiertes Wasser | q.s. |

Herstellung: Die festen Ingredienzien werden zunächst durch ein Sieb mit 0,6 mm Maschenweite getrieben. Dann werden Wirkstoff, Lactose, Talkum, Magnesiumstearat und die Hälfte der Stärke innig vermischt. Die andere Hälfte der Stärke wird in 65 ml Wasser suspendiert und diese Suspension zu einer siedenden Lösung des Polyäthylenglykols in 260 ml

Wasser hinzugegeben und das Gemisch, wenn nötig unter Hinzufügen von Wasser, granuliert. Das Granulat wird über Nacht bei 35° getrocknet, durch ein Sieb mit 1,2 mm Maschenweite getrieben und zu beidseitig konkaven Tabletten von etwa 6 mm Durchmesser verpresst.

In analoger Weise können auch Tabletten enthaltend jeweils 100 mg einer der in den Beispielen 1, 2 und 4 bis 11 genannten Verbindungen der Formel I hergestellt werden, wobei diese Verbindungen auch in Form von Salzen vorliegen können.

Patentansprüche für die Vertragsstaaten: BE, CH, LI, DE, FR, GB, IT, LU, NL, SE

1. Neue 1,2,4-Triazacycloalkadienderivate der Formel

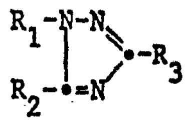

(I),

worin die Reste $R_1$ und $R_2$ unabhängig voneinander unsubstituierte oder durch aliphatische Kohlenwasserstoffreste, freies, veräthertes oder verestertes Hydroxy, gegebenenfalls S-oxidiertes veräthertes Mercapto, freies oder aliphatisch substituiertes Amino, Nitro und/oder Trifluormethyl substituierte Aryl- oder ggf. N-oxidierte Heteroarylgruppen bedeuten und $R_3$ einen unsubstituierten, mindestens 2 C-Atome aufweisenden oder durch eine Cyanogruppe, eine ggf. veresterte oder amidierte Carboxygruppe, eine oder zwei ggf. veresterte oder verätherte Hydroxygruppe(n), eine oder zwei verätherte Mercaptogruppe(n), eine oxidierte verätherte Mercaptogruppe oder eine Oxogruppe substituierten, ggf. durch Thia oder Oxa unterbrochenen aliphatischen oder einen unsubstituierten cycloaliphatischen Kohlenwasserstoffrest darstellt, und ihre Salze.

2. Verbindungen gemäss Anspruch 1 der Formel I, worin die Reste $R_1$ und $R_2$ unabhängig voneinander unsubstituierte oder durch Niederalkyl, Niederalkoxy bzw. an vicinalen C-Atomen Niederalkyl(id)endioxy, Hydroxy, Halogen, Niederalkanoyloxy, aber auch unsubstituiertes oder durch Niederalkyl, Niederalkoxy und/oder Halogen substituiertes Benzoyloxy, Niederalkylthio, Niederalkansulfinyl, Niederalkansulfonyl, Amino, Mono- oder Diniederalkylamino, ferner 4- bis 7-gliedriges Alkylen- bzw. 3-Aza-, 3-Oxa- oder 3-Thianiederalkylenamino, Nitro und/oder Trifluormethyl substituierte 6 bis und mit 10 C-Atomen aufweisende Aryl- oder ein Sauerstoff- oder Schwefelatom und gegebenenfalls zusätzlich ein Stickstoffatom aufweisende 5-gliedrige bzw. gegebenenfalls N-oxidierte ein oder zwei Stickstoffatom(e) aufweisende 6-gliedrige Heteroarylreste bedeuten und $R_3$ Nieder-

alkyl mit mindestens 2 C-Atomen, Mono- oder Dihydroxyniederalkyl, Mono- oder Diniederalkanoyloxyniederalkyl, Hydroxyniederalkoxyniederalkyl, Niederalkoxyniederniederalkylthioniederalkyl, Mono- oder Diniederalkoxyniederalkyl, Niederalkylendioxyniederalkyl, Niederalkylendithioniederalkyl, Mono- oder Diniederalkylthioniederalkyl, Niederalkansulfinylniederalkyl, Niederalkansulfonylniederalkyl, Oxoniederalkyl, Carboxyniederalkyl, Niederalkoxycarbonylniederalkyl, Cyanoniederalkyl, Carbamylniederalkyl, N-Mono- oder N,N-Diniederalkylcarbamylniederalkyl, ferner 4- bis 7-gliedriges N,N-Niederalkylen- bzw. N,N-(3-Aza-, 3-Oxa- oder 3-Thia)-niederalkylencarbamylniederalkyl darstellt, und ihre Salze.

3. Verbindungen gemäss Anspruch 1 der Formel I, worin die Reste $R_1$ und $R_2$ unabhängig voneinander unsubstituiertes oder durch Niederalkoxy mit bis und mit 4 C-Atomen, Niederalkylthio mit bis und mit 4 C-Atomen oder Halogen der Atomnummer bis und mit 35 substituiertes Phenyl, gegebenenfalls N-oxidiertes Pyridyl oder unsubstituiertes Thienyl bedeuten und $R_3$ über ein sekundäres oder tertiäres C-Atom gebundenes Niederalkyl bis 3 bis und mit 7 C-Atomen, die Cyano- oder Niederalkylthiogruppe in $\alpha$-Stellung tragendes Cyanoniederalkyl mit bis und mit 4 C-Atomen im Niederalkyltheil oder Niederalkylthioniederalkyl mit jeweils bis und mit 4 C-Atomen bedeutet oder Hydroxyniederalkyl mit bis und mit 4 C-Atomen, Niederalkanoyloxyniederalkyl mit bis und mit 7 C-Atomen im Niederalkanoyloxyteil und bis und mit 4 C-Atomen im Niederalkylteil, Oxoniederalkyl mit bis und mit 4 C-Atomen oder Diniederalkoxyniederalkyl bzw. Niederalkylendioxyniederalkyl mit jeweils bis und mit 4 C-Atomen in jedem Alkyl(en)teil bedeutet, und ihre Salze.

4. Verbindungen gemäss Anspruch 1 der Formel I, worin $R_1$ und $R_2$ durch Niederalkoxy mit bis und mit 4 C-Atomen substituiertes Phenyl bedeuten oder $R_1$ Pyridyl und $R_2$ Phenyl darstellt und $R_3$ über ein tertiäres C-Atom gebundenes

Niederalkyl mit bis und mit 7 C-Atomen, die Carboxy-, Cyano-, Niederalkoxycarbonyl-, Niederalkylthio-, Hydroxyniederalkylthio-, Oxoniederalkylthio-, Diniederalkoxyniederalkylthio bzw. Niederalkylendioxyniederalkylthiogruppe in α-Stellung tragendes Carboxyniederalkyl, Cyanoniederalkyl, Niederalkoxycarbonylniederalkyl, Niederalkylthioniederalkyl, ω-Hydroxyniederalkylthioniederalkyl, ω-Oxoniederalkylthioniederalkyl, Diniederalkoxyniederalkylthioniederalkyl oder Niederalkylendioxyniederalkylthioniederalkyl mit insgesamt bis und mit 7 C-Atomen oder Hydroxyniederalkyl mit bis und mit 7 C-Atomen bedeutet, und ihre Salze.

5. Verbindungen gemäss Anspruch 1 der Formel I, worin $R_1$ und $R_2$ durch Niederalkoxy mit bis und mit 4 C-Atomen substituiertes Phenyl bedeuten und $R_3$ über ein tertiäres C-Atom gebundenes Niederalkyl mit bis und mit 7 C-Atomen, die Cyano-, Niederalkylthio- bzw. Diniederalkoxyniederalkylthiogruppe in α-Stellung tragendes Cyanoniederalkyl mit insgesamt bis und mit 7 C-Atomen, Niederalkylthioniederalkyl mit insgesamt bis und mit 7 C-Atomen, Diniederalkoxyniederalkylthioniederalkyl mit insgesamt bis und mit 7 C-Atomen oder Hydroxyniederalkyl mit bis und mit 7 C-Atomen bedeutet, und ihre Salze.

6. 1,5-Bis(p-methoxyphenyl)-3-tertiärbutyl-1H-1,2,4-triazol oder ein Salz davon.

7. 5-Phenyl-1-(3-pyridyl)-3-tertiärbutyl-1H-1,2,4-triazol oder ein Salz davon.

8. 3-Aethylthiomethyl-1,5-bis(p-methoxyphenyl)-1H-1,2,4-triazol oder ein Salz davon.

9. 2-[1,5-Bis(p-methoxyphenyl)-1H-1,2,4-triazol-3]-propan-2-ol oder ein Salz davon.

10. 1-Phenyl-5-(3-pyridyl)-3-tertiärbutyl-1H-1,2,4-triazol, 5-(p-Methoxyphenyl)-1-(3-pyridyl)-3-tertiärbutyl-1H-1,2,4-triazol, 1,5-Bis(p-methoxyphenyl)-1H-1,2,4-triazol-3-methanol, 2-[1,5-Bis(p-methoxyphenyl)-1H-1,2,4-triazol-3]-acetonitril, 2-[1,5-Bis(p-methoxyphenyl)-1H-1,2,4-triazol-3]-2-methyl-propionitril, 2-[1,5-Bis(p-methoxyphenyl)-1H-1,2,4-triazol-3]-2-methyl-propanol oder 1-[1,5-Bis(p-methoxyphenyl)-1H-1,2,4-triazol-3-ylmethylthio]-2,2-dimethoxy-äthan oder jeweils ein Salz davon.

11. Pharmazeutische Präparate enthaltend eine Verbindung gemäss einem der Ansprüche 1 - 10 neben üblichen pharmazeutischen Hilfs- und/oder Trägerstoffen.

12. Verfahren zur Herstellung neuer 1,2,4-Triazacycloalkadienderivate der Formel

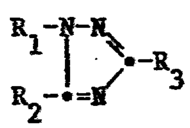 (I),

worin die Reste $R_1$ und $R_2$ unabhängig voneinander unsubstituierte oder durch aliphatische Kohlenwasserstoffreste, freies, veräthertes oder verestertes Hydroxy, gegebenenfalls S-oxidiertes veräthertes Mercapto, freies oder aliphatisch substituiertes Amino, Nitro und/oder Trifluormethyl substituierte Aryl- oder ggf. N-oxidierte Heteroarylgruppen bedeuten und $R_3$ einen unsubstituierten, mindestens 2 C-Atome aufweisenden oder durch eine Cyanogruppe, eine ggf. veresterte oder amidierte Carboxygruppe, eine oder zwei ggf. veresterte oder verätherte Hydroxygruppe(n), eine oder zwei verätherte Mercaptogruppe(n), eine oxidierte verätherte Mercaptogruppe oder eine Oxogruppe substituierten, ggf. durch Thia oder Oxa unterbrochenen aliphatischen oder einen unsubstituierten cycloaliphatischen Kohlenwasserstoffrest darstellt, und ihrer Salze, dadurch gekennzeichnet, dass man aus einer Verbindung der Formel

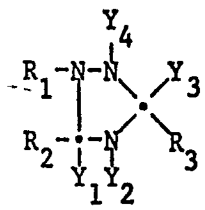

(II),

worin einer der Reste $Y_1$ und $Y_2$ einen abspaltbaren Rest Y und der andere ein Wasserstoffatom bedeutet und $Y_3$ und $Y_4$ gemeinsam eine zusätzliche Bindung darstellen, oder worin $Y_4$ einen abspaltbaren Rest Y und $Y_3$ Wasserstoff bedeutet und $Y_1$ und $Y_2$ eine zusätzliche Bindung darstellen, oder aus einem Salz davon unter Einführung einer zusätzliche Bindung HY abspaltet oder in einer Verbindung der Formel

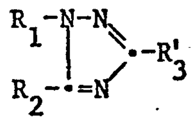

(XVIII),

worin $R'_3$ einen in eine Gruppe $R_3$ überführbaren Rest bedeutet, $R'_3$ in die gewünschte Gruppe $R_3$ überführt oder eine Verbindung der Formel

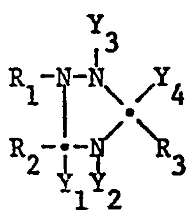

(II),

worin $Y_1$ und $Y_2$ Wasserstoff und $Y_3$ und $Y_4$ eine zusätzliche Bindung oder Wasserstoff oder $Y_1$ und $Y_2$ eine zusätzliche Bindung und $Y_3$ und $Y_4$ Wasserstoff bedeuten, dehydriert, erforderlichenfalls ein verfahrensgemäss erhältliches Isomerengemisch in die Komponenten auftrennt und das Isomere der Formel I isoliert und gewünschtenfalls eine verfahrensgemäss erhältliche Verbindung in eine andere Verbindung der Formel I umwandelt und/oder eine verfahrensgemäss erhältliche freie Verbindung in ein Salz oder ein verfahrensgemäss erhältliches Salz in die freie Verbindung oder in ein anderes Salz überführt.

13. Eine Verbindung gemäss einer der Ansprüche 1 - 10 zur Anwendung in einem Verfahren zur therapeutischen Behandlung des menschlichen oder tierischen Körpers.

14. Eine Verbindung gemäss einer der Ansprüche 1 - 10 zur Anwendung gemäss Anspruch 13 als Antiinflammatorikum und/oder Analgetikum bzw. zur Herstellung eines solchen auf nicht-chemischem Wege.

15. Pharmazeutische Präparate enthaltend eine Verbindung gemäss einem der Ansprüche 2, 3 und 6-10 neben üblichen pharmazeutischen Hilfs- und Trägerstoffen.

16. Das Verfahren der Beispiele 1 bis 12.

17. Die gemäss dem Verfahren eines der Ansprüche 12 und 16 erhältlichen Verbindungen, verwendeten Ausgangsstoffe und gebildeten Zwischenprodukte sowie Verfahren zur Herstellung dieser Ausgangsstoffe und Zwischenprodukte.

FO 7.4 KVB/hc*

Patentansprüche für den Vertragsstaat: AT

1. Verfahren zur Herstellung neuer 1,2,4-Triazacycloalkadienderivate der Formel

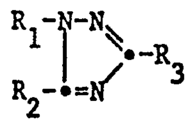

(I),

worin die Reste $R_1$ und $R_2$ unabhängig voneinander unsubstituierte oder durch aliphatische Kohlenwasserstoffreste, freies, veräthertes oder verestertes Hydroxy, gegebenenfalls S-oxidiertes veräthertes Mercapto, freies oder aliphatisch substituiertes Amino, Nitro und/oder Trifluormethyl substituierte Aryl- oder ggf. N-oxidierte Heteroarylgruppen bedeuten und $R_3$ einen unsubstituierten, mindestens 2 C-Atome aufweisenden oder durch eine Cyanogruppe, eine ggf. veresterte oder amidierte Carboxygruppe, eine oder zwei ggf. veresterte oder verätherte Hydroxygruppe(n), eine oder zwei verätherte Mercaptogruppe(n), eine oxidierte verätherte Mercaptogruppe oder eine Oxogruppe substituierten, ggf. durch Thia oder Oxa unterbrochenen aliphatischen oder einen unsubstituierten cycloaliphatischen Kohlenwasserstoffrest darstellt, und ihrer Salze, dadurch gekennzeichnet, dass man aus einer Verbindung der Formel

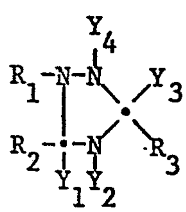

(II),

worin einer der Reste $Y_1$ und $Y_2$ einen abspaltbaren Rest Y und der andere ein Wasserstoffatom bedeutet und $Y_3$ und $Y_4$ gemeinsam eine zusätzliche Bindung darstellen, oder worin $Y_4$ einen abspaltbaren Rest Y und $Y_3$ Wasserstoff bedeutet und $Y_1$ und $Y_2$ eine zusätzliche Bindung darstellen, oder aus einem Salz davon unter Einführung einer zusätzliche Bindung HY abspaltet oder in einer Verbindung der Formel

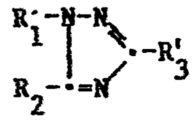

(XVIII),

worin $R'_3$ einen in eine Gruppe $R_3$ überführbaren Rest bedeutet, $R'_3$ in die gewünschte Gruppe $R_3$ überführt oder eine Verbindung der Formel

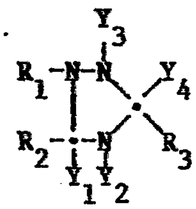

(II),

worin $Y_1$ und $Y_2$ Wasserstoff und $Y_3$ und $Y_4$ eine zusätzliche Bindung oder Wasserstoff oder $Y_1$ und $Y_2$ eine zusätzliche Bindung und $Y_3$ und $Y_4$ Wasserstoff bedeuten, dehydriert, erforderlichenfalls ein verfahrensgemäss erhältliches Isomerengemisch in die Komponenten auftrennt und das Isomere der Formel I isoliert und gewünschtenfalls eine verfahrensgemäss erhältliche Verbindung in eine andere Verbindung der Formel I umwandelt und/oder eine verfahrensgemäss erhältliche freie Verbindung in ein Salz oder ein verfahrensgemäss erhältliches Salz in die freie Verbindung oder in ein anderes Salz überführt.

2. Verfahren gemäss Anspruch 1, dadurch gekennzeichnet, dass man Verbindungen der Formel I, worin die Reste $R_1$ und $R_2$ unabhängig voneinander unsubstituierte oder durch Niederalkyl, Niederalkoxy bzw. an vicinalen C-Atomen Niederalkyl(id)endioxy, Hydroxy, Halogen, Niederalkanoyloxy, aber auch unsubstituiertes oder durch Niederalkyl, Niederalkoxy und/oder Halogen substituiertes Benzoyloxy, Niederalkylthio, Niederalkansulfinyl, Niederalkansulfonyl, Amino, Mono- oder Diniederalkylamino, ferner 4- bis 7-gliedriges Alkylen- bzw. 3-Aza-, 3-Oxa- oder 3-Thianiederalkylenamino, Nitro und/oder Trifluormethyl substituierte 6 bis und mit 10 C-Atomen aufweisende Aryl- oder ein Sauerstoff- oder Schwefelatom und gegebenenfalls zusätzlich ein Stickstoffatom aufweisende 5-gliedrige bzw. gegebenenfalls N-oxidierte ein oder zwei Stickstoffatom(e) aufweisende 6-gliedrige Heteroarylreste bedeuten und $R_3$ Nieder-

alkyl mit mindestens 2 C-Atomen, Mono- oder Dihydroxyniederalkyl, Mono- oder Diniederalkanoyloxyniederalkyl, Hydroxyniederalkoxyniederalkyl, Niederalkoxyniederniederalkylthioniederalkyl, Mono- oder Diniederalkoxyniederalkyl, Niederalkylendioxyniederalkyl, Niederalkylendithioniederalkyl, Mono- oder Diniederalkylthioniederalkyl, Niederalkansulfinylniederalkyl, Niederalkansulfonylniederalkyl, Oxoniederalkyl, Carboxyniederalkyl, Niederalkoxycarbonylniederalkyl, Cyanoniederalkyl, Carbamylniederalkyl, N-Mono- oder N,N-Diniederalkylcarbamylniederalkyl, ferner 4- bis 7-gliedriges N,N-Niederalkylen- bzw. N,N-(3-Aza-, 3-Oxa- oder 3-Thia)-niederalkylencarbamylniederalkyl darstellt, oder ihre Salze herstellt.

3. Verfahren gemäss Anspruch 1, dadurch gekennzeichnet, dass man Verbindungen der Formel I, worin die Reste $R_1$ und $R_2$ unabhängig voneinander unsubstituiertes oder durch Niederalkoxy mit bis und mit 4 C-Atomen, Niederalkylthio mit bis und mit 4 C-Atomen oder Halogen der Atomnummer bis und mit 35 substituiertes Phenyl, gegebenenfalls N-oxidiertes Pyridyl oder unsubstituiertes Thienyl bedeuten und $R_3$ über ein sekundäres oder tertiäres C-Atom gebundenes Niederalkyl bis 3 bis und mit 7 C-Atomen, die Cyano- oder Niederalkylthiogruppe in α-Stellung tragendes Cyanoniederalkyl mit bis und mit 4 C-Atomen im Niederalkyltheil oder Niederalkylthioniederalkyl mit jeweils bis und mit 4 C-Atomen bedeutet oder Hydroxyniederalkyl mit bis und mit 4 C-Atomen, Niederalkanoyloxyniederalkyl mit bis und mit 7 C-Atomen im Niederalkanoyloxyteil und bis und mit 4 C-Atomen im Niederalkylteil, Oxoniederalkyl mit bis und mit 4 C-Atomen oder Diniederalkoxyniederalkyl bzw. Niederalkylendioxyniederalkyl mit jeweils bis und mit 4 C-Atomen in jedem Alkyl(en)teil bedeutet, oder ihre Salze herstellt.

4. Verfahren gemäss Anspruch 1, dadurch gekennzeichnet, dass man Verbindungen der Formel I, worin $R_1$ und $R_2$ durch Niederalkoxy mit bis und mit 4 C-Atomen substituiertes Phenyl bedeuten oder $R_1$ Pyridyl und $R_2$ Phenyl darstellt und $R_3$ über ein tertiäres C-Atom gebundenes Niederalkyl mit bis und mit 7 C-Atomen, die Carboxy-, Cyano-, Niederalkoxycarbonyl-, Niederalkylthio-, Hydroxyniederalkylthio-, Oxoniederalkylthio-, Diniederalkoxyniederalkylthio bzw. Niederalkylendioxyniederalkylthiogruppe in α-Stellung tragendes Carboxyniederalkyl, Cyanoniederalkyl, Niederalkoxycarbonylniederalkyl, Niederalkylthioniederalkyl, ω-Hydroxyniederalkylthioniederalkyl, ω-Oxoniederalkylthioniederalkyl, Diniederalkoxyniederalkylthioniederalkyl oder Niederalkylendioxyniederalkylthioniederalkyl mit insgesamt bis und mit 7 C-Atomen oder Hydroxyniederalkyl mit bis und mit 7 C-Atomen bedeutet, oder ihre Salze herstellt.

5. Verfahren gemäss Anspruch 1, dadurch gekennzeichnet, dass man Verbindungen der Formel I, worin $R_1$ und $R_2$ durch Niederalkoxy mit bis und mit 4 C-Atomen substituiertes Phenyl bedeuten und $R_3$ über ein tertiäres C-Atom gebundenes Niederalkyl mit bis und mit 7 C-Atomen, die Cyano-, Niederalkylthio- bzw. Diniederalkoxyniederalkylthiogruppe in α-Stellung tragendes Cyanoniederalkyl mit insgesamt bis und mit 7 C-Atomen, Niederalkylthioniederalkyl mit insgesamt bis und mit 7 C-Atomen, Diniederalkoxyniederalkylthioniederalkyl mit insgesamt bis und mit 7 C-Atomen oder Hydroxyniederalkyl mit bis und mit 7 C-Atomen bedeutet, oder ihre Salze herstellt.

6. Verfahren gemäss Anspruch 1, dadurch gekennzeichnet, dass man 1,5-Bis(p-methoxyphenyl)-3-tertiärbutyl-1H-1,2,4-triazol oder ein Salz davon herstellt.

7. Verfahren gemäss Anspruch 1, dadurch gekennzeichnet, dass man 5-Phenyl-1-(3-pyridyl)-3-tertiärbutyl-1H-1,2,4-triazol oder ein Salz davon herstellt.

8. Verfahren gemäss Anspruch 1, dadurch gekennzeichnet, dass man 3-Aethylthiomethyl-1,5-bis(p-methoxyphenyl)-1H-1,2,4-triazol oder ein Salz davon herstellt.

9. Verfahren gemäss Anspruch 1, dadurch gekennzeichnet, dass man 2-[1,5-Bis(p-methoxyphenyl)-1H-1,2,4-triazol-3]-propan-2-ol oder ein Salz davon herstellt.

10. Verfahren gemäss Anspruch 1, dadurch gekennzeichnet, dass man 1-Phenyl-5-(3-pyridyl)-3-tertiärbutyl-1H-1,2,4-triazol, 5-(p-Methoxyphenyl)-1-(3-pyridyl)-3-tertiärbutyl-1H-1,2,4-triazol, 1,5-Bis(p-methoxyphenyl)-1H-1,2,4-triazol-3-methanol, 2-[1,5-Bis(p-methoxyphenyl)-1H-1,2,4-triazol-3]-acetonitril, 2-[1,5-Bis(p-methoxyphenyl)-1H-1,2,4-triazol-3]-2-methyl-propionitril, 2-[1,5-Bis(p-methoxyphenyl)-1H-1,2,4-triazol-3]-2-methyl-propanol oder 1-[1,5-Bis(p-methoxyphenyl)-1H-1,2,4-triazol-3-ylmethylthio]-2,2-dimethoxy-äthan oder jeweils ein Salz davon.

11. Das Verfahren der Beispiele 1 bis 12.

12. Verfahren zur Herstellung pharmazeutischer Präparate, dadurch gekennzeichnet, dass man eine Verbindung gemäss einem der Ansprüche 1 - 10 mit üblichen pharmazeutischen Hilfs- und/oder Trägestoffen vermischt.

FO 7.4 KVB/hc*/gb*

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

0162217

Nummer der Anmeldung

EP 85 10 3011.4

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| A | EP-A1-0 051 084 (AMERICAN CYANAMID CO.)<br>* Ansprüche 1,5 *<br>-- | 1,11 | C07D249/08<br>C07D401/04<br>A61KK31/41 |
| A | EP-A1-0 070 089 (KUREHA KAGAKU KOGYO K.K.)<br>* Anspruch 1 *<br>-- | 1 | |
| P,A | CHEMICAL ABSTRACTS, Bd. 100, Nr. 13, 26. März 1984, Columbus, Ohio, US; D.MODERHACK:"3-Phenyl-1,2,4-triazole-5-carbaldehydes substituted in position 1"; Seite 645, Zusammenfassung Nr. 103265c<br>* Formel V * | 1 | |
| A | & Liebigs Ann. Chem.., Nr. 1, 1984, Seiten 48-65<br>---- | | |
| | | | **RECHERCHIERTE SACHGEBIETE (Int. Cl. ³)** |
| | | | C07D249/00<br>C07D401/00 |

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-15

Unvollständig recherchierte Patentansprüche: ---

Nicht recherchierte Patentansprüche: 16,17

Grund für die Beschränkung der Recherche: (siehe Regel 29(6) EPÜ sowie die Entscheidung Nr. T 150/82 der Technischen Beschwerdekammer 3.3.1 vom 07.02.1984)

| Recherchenort | Abschlußdatum der Recherche | Prüfer |
|---|---|---|
| Berlin | 13-06-1985 | HAß |

**KATEGORIE DER GENANNTEN DOKUMENTEN**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1505.1 03.82